# EUROPÄISCHE PATENTSCHRIFT

(11) **EP 2 810 597 B1**
(45) Veröffentlichungstag und Bekanntmachung des Hinweises auf die Patenterteilung: **03.08.2016**
(21) Anmeldenummer: 14169280.6
(22) Anmeldetag: 21.05.2014
(51) Int. Cl.: A61B 1/00, A61B 17/34

(54) **Vorrichtung zum Bereitstellen einer Zugriffsöffnung in einen Körper, insbesondere für eine Wirbelsäulenoperation**
Device for providing an access port into a body, in particular for spinal surgery
Dispositif de préparation d'une ouverture d'accès dans un corps, en particulier pour une opération à la colonne vertébrale

(30) Priorität: 22.05.2013 DE 102013209413
(43) Veröffentlichungstag der Anmeldung: 10.12.2014
(73) Patentinhaber: Karl Storz GmbH & Co. KG, 78532 Tuttlingen (DE)
(72) Erfinder: Oberländer, Martin, 78234 Engen (DE); Stark, Roland E., 88690 Uhldingen-Mühlhofen (DE)
(74) Vertreter: RLTG

(56) Entgegenhaltungen:
- EP-A1- 1 192 905
- EP-A1- 1 649 823
- EP-B1- 1 466 564
- US-A1- 2003 055 437

## Beschreibung

Die vorliegende Erfindung betrifft eine Vorrichtung zum Bereitstellen einer Zugriffsöffnung in einen Körper, insbesondere für eine Wirbelsäulenoperation, umfassend einen entlang einer Vorrichtungskörperlängsachse sich erstreckenden, rohrartigen Vorrichtungskörper mit einem innerhalb eines Körpers zu positionierenden distalen Ende und einem außerhalb eines Körpers zu positionierenden proximalen Ende sowie eine Optikträgeranordnung, wobei die Optikträgeranordnung im Bereich des proximalen Endes mit einem auf einer Bewegungsbahn um die Vorrichtungskörperlängsachse bewegbaren Optikträger positioniert oder positionierbar ist.

Eine derartige Vorrichtung zur Durchführung perkutaner Operationen, beispielsweise im Lendenwirbelbereich oder im Halswirbelbereich eines menschlichen Körpers, ist aus der EP 1 466 564 B1 bekannt. Diese bekannte Vorrichtung umfasst einen im Wesentlichen zylindrischen, längs einer Vorrichtungskörperlängsachse langgestreckten Vorrichtungskörper. In Abstand zum proximalen Ende dieses Vorrichtungskörpers ist an diesem ein seitlich davon sich weg erstreckender, armartiger Träger zur Befestigung des Vorrichtungskörpers an einem Gelenkstativ oder dergleichen angebracht. Um an dem Vorrichtungskörper ein Optiksystem anbringen zu können, wird ein hülsenartiger Optikträger auf den Vorrichtungskörper an dessen proximalen Ende aufgeschoben, so dass dieser Optikträger sich im Wesentlichen zwischen dem vom Vorrichtungskörper seitlich sich weg erstreckenden, armartigen Träger und dem proximalen Ende des Vorrichtungskörpers erstreckt. Der Optikträger kann mit dem daran fixierten Optiksystem um eine Vorrichtungskörperlängsachse herum auf einer im Wesentlichen kreisförmigen Bewegungsbahn bewegt werden.

Genau wie EP 1 466 564 B1 offenbart auch die EP 1 192 905 A1 die Merkmale des Oberbegriffs des 1. Anspruchs.

Die EP 1 649 823 A1 offenbart ein System zur Behandlung eines Gewebes, bei welchem ein plattenartiger Halter an einer Außenseite eines zu behandelnden Körpers auf der Haut aufliegend positioniert wird. An dem plattenartigen Halter ist ein gleichermaßen außerhalb des zu behandelnden Körpers zu positionierendes rohrartiges Bauteil vorgesehen, welches bezüglich des plattenartigen Halters um seine Längsachse gedreht werden kann. An einem proximalen Endbereich dieses rohrartigen Bauteils kann eine Magnetsensoranordnung positioniert werden, deren Ausgangssignal in Verbindung mit dem Ausgangssignal einer Ultraschallsensoranordnung Information über die Positionierung der Vorrichtung liefert.

Es ist die Aufgabe der vorliegenden Erfindung, eine Vorrichtung zum Bereitstellen einer Zugriffsöffnung in einen Körper, insbesondere zur Durchführung einer Wirbelsäulenoperation, bereitzustellen, welche bei leicht handzuhabendem und funktionssicherem Aufbau eine Positionierung des proximalen Endes des Vorrichtungskörpers und somit des Optikträgers und des daran fixierten Optiksystems nahe an einer Körperoberfläche ermöglicht.

Erfindungsgemäß wird diese Aufgabe gelöst durch eine Vorrichtung zum Bereitstellen einer Zugriffsöffnung in einen Körper, insbesondere für eine Wirbelsäulenoperation, umfassend einen entlang einer Vorrichtungskörperlängsachse sich erstreckenden, rohrartigen Vorrichtungskörper mit einem innerhalb eines Körpers zu positionierenden distalen Ende und einem außerhalb eines Körpers zu positionierenden proximalen Ende sowie eine Optikträgeranordnung, wobei die Optikträgeranordnung im Bereich des proximalen Endes mit einem auf einer Bewegungsbahn um die Vorrichtungskörperlängsachse bewegbaren Optikträger positioniert oder positionierbar ist.

Dabei ist weiter vorgesehen, dass die Optikträgeranordnung ein den Optikträger tragendes Führungsbahnelement mit einem durch Verschwenkung um eine Schwenkachse in einer Betriebsstellung im Bereich des proximalen Endes des Vorrichtungskörpers positionierbaren Führungsbahnbereich umfasst.

Während bei dem aus dem Stand der Technik bekannten Aufbau der Optikträger im Bereich des proximalen Endes des Vorrichtungskörpers auf diesen aufgeschoben wird und somit dieser vom Optikträger übergriffene Längenbereich des Vorrichtungskörpers außerhalb des zu behandelnden Körpers verbleiben muss, ist bei dem erfindungsgemäßen Aufbau ein Führungsbahnelement vorgesehen, an welchem der Optikträger zur Bewegung um die Vorrichtungskörperlängsachse getragen ist. Dieses Führungsbahnelement wird vermittels einer Schwenkbewegung anschließend an das proximale Ende des Vorrichtungskörpers positioniert, also nicht notwendigerweise über einen größeren Längenbereich des Vorrichtungskörpers im Bereich des proximalen Endes desselben geschoben. Dies gestattet es, den Vorrichtungskörper tiefer in den zu behandelnden Körper eintauchen zu lassen bzw. den während eines operativen Eingriffs außerhalb des behandelten Körpers verbleibenden Bereich des Vorrichtungskörpers kürzer zu gestalten. Damit rückt der Optikträger und somit auch das an diesem getragene Optiksystem näher an die Körperoberfläche, also die Haut, des behandelten Körpers heran.

Eine definierte Bewegung des Optikträgers an dem diesen tragenden Führungsbahnelement kann dadurch gewährleistet werden, dass der Führungsbahnbereich ringartig ausgebildet ist und eine ringartige erste Führungsformation, vorzugsweise Führungsnut oder Führungsfläche, aufweist, wobei eine zweite Führungsformation, vorzugsweise Führungsvorsprung oder Führungsring, des Optikträgers zum Bewegen des Optikträgers auf seiner Bewegungsbahn um die Vorrichtungskörperlängsachse entlang der ringartigen ersten Führungsformation geführt ist und an vorzugsweise beliebiger Positionierung entlang der ersten Führungsformation fixierbar ist.

Um bei dem Führungsbahnelement beide wesentliche Funktionalitäten, nämlich zum einen das Tragen des Optikträgers und zum anderen die schwenkbare Anbringung bezüglich des Vorrichtungskörpers, gewährleisten zu können, wird vorgeschlagen, dass das Führungsbahnelement ferner einen Schwenk/Arretierbereich aufweist zur schwenkbaren Anbringung und Arretierung des Führungsbahnelements in der Betriebsstellung an einem von dem Vorrichtungskörper sich weg erstreckenden Schwenkträgerelement. Das Schwenkträgerelement kann des Weiteren genutzt werden, um den Vorrichtungskörper an einem Gelenkstativ oder dergleichen anzubringen.

Zur festen Verbindung des Schwenkträgerelements mit dem Vorrichtungskörper wird vorgeschlagen, dass das Schwenkträgerelement einen den Vorrichtungskörper vorzugsweise ringartig umgebenden und vorzugsweise mit dem proximalen Ende des Vorrichtungskörpers bündig abschließenden Befestigungbereich und einen von dem Befestigungsbereich sich weg erstreckenden Trägerbereich umfasst. Die Positionierung des Schwenkträgerelements sehr nahe am proximalen Ende des Vorrichtungskörpers, nämlich derart, dass dessen Befestigungsbereich bündig abschließend mit dem proximalen Ende bzw. einer Stirnfläche des Vorrichtungskörpers am proximalen Ende liegt, wird bei dem erfindungsgemäßen Aufbau dadurch möglich, dass der Optikträger an sich nicht auf den Vorrichtungskörper im Bereich des proximalen Endes desselben aufzuschieben ist, sondern dass die Optikträgeranordnung eine zum Tragen des Optikträgers speziell vorgesehene Baugruppe, nämlich das Führungsbahnelement, umfasst, deren Führungsbahnbereich in Richtung der Vorrichtungskörperlängsachse axial angrenzend an das proximale Ende des Vorrichtungskörpers positioniert werden kann. Es ist darauf hinzuweisen, dass die Ausgestaltung des Befestigungsbereichs derart, dass dieser den Vorrichtungskörper ringartig umgibt, besonders vorteilhaft ist, da somit bei einer Positionierung sehr nahe am proximalen Ende des Vorrichtungskörpers eine stabile Halterung, beispielsweise durch Verschweißung, durch Verklebung oder in sonstiger materialschlüssiger Weise, erreicht werden kann. Alternativ wäre auch das Bereitstellen des Befestigungsbereichs als Ende eines stabartig ausgebildeten Schwenkträgerelements und das Festlegen dieses Endes an einem Außenoberflächenbereich des Vorrichtungskörpers beispielsweise durch Verschweißung oder Verklebung möglich.

Um eine definierte Schwenkbewegung des Führungsbahnelements bezüglich des Vorrichtungskörpers bzw. des am Vorrichtungskörper vorgesehenen Schwenkträgerelements zu gewährleisten, wird vorgeschlagen, dass an einer Baugruppe von Schwenkträgerelement und Schwenk/Arretierbereich, vorzugsweise dem Schwenkträgerelement, wenigstens ein Schwenkvorsprung vorgesehen ist und dass an der anderen Baugruppe von Schwenkträgerelement und Schwenk/Arretierbereich, vorzugsweise dem Schwenk/Arretierbereich des Führungsbahnelements, in Zuordnung zu jedem Schwenkvorsprung eine zum Einführen eines Schwenkvorsprungs in einem Umfangsbereich offene Schwenkaussparung vorgesehen ist. Es ist hier darauf hinzuweisen, dass im Sinne der vorliegenden Erfindung eine Baugruppe zwar mehrere Bauteile umfassen kann bzw. aus mehreren Bauteilen zusammengesetzt sein kann, grundsätzlich jedoch auch eine einstückig ausgebildete Komponente der Vorrichtung umfassen kann.

Da es vorteilhaft ist, während des Vorgangs zum Einführen des Vorrichtungskörpers in einen in dem zu behandelnden Körper gebildeten Einschnitt das Führungsbahnelement mit dem daran getragenen Optikträger vom Vorrichtungskörper bzw. vom Schwenkträgerelement losgelöst zu haben, gleichwohl jedoch nach dem Anbringen des Führungsbahnelements am Schwenkträgerelement eine gegen Loslösen gesicherte Relativpositionierung dieser beiden Baugruppen bezüglich einander gewährleistet ist, wird weiter vorgeschlagen, dass der wenigstens eine Schwenkvorsprung zum Einführen in eine Schwenkaussparung in einer Einführrelativpositionierung zwischen dem Schwenk/Arretierbereich und dem Schwenkträgerelement in wenigstens einem Umfangsbereich eine geringere Querschnittsabmessung aufweist, als in einem anderen Umfangsbereich, wobei bei in einer Schwenkaussparung aufgenommenem Schwenkvorsprung und aus der Einführrelativpositionierung bezüglich einander verschwenktem Schwenk/Arretierbereich und Schwenkträgerelement der wenigstens eine Schwenkvorsprung nicht aus der Schwenkaussparung herausbewegbar ist.

Eine definierte Relativpositionierung des Führungsbahnelements bezüglich des Schwenkträgerelements bei bzw. auch nach Durchführung der Schwenkbewegung kann dadurch weiter unterstützt werden, dass an dem Schwenkträgerelement zwei sich im Wesentlichen entlang einer gemeinsamen Schwenkachse erstreckende Schwenkvorsprünge vorgesehen sind und dass der Schwenk/Arretierbereich des Führungsbahnelements gabelartig mit zwei Gabelendbereichen ausgebildet ist und in jedem Gabelendbereich eine Schwenkaussparung aufweist.

Da während der Durchführung eines operativen Eingriffs eine stabile und gegen ungewünschte Bewegung gesicherte Positionierung des Führungsbahnelements bezüglich des Vorrichtungskörpers erforderlich ist, wird weiter vorgeschlagen, dass an einer Baugruppe von Schwenkträgerelement und Schwenk/Arretierbereich, vorzugsweise dem Schwenkträgerelement, wenigstens ein Arretiervorsprung vorgesehen ist und dass an der anderen Baugruppe von Schwenkträgerelement und Schwenk/Arretierbereich, vorzugsweise dem Schwenk/Arretierbereich des Führungsbahnelements, in Zuordnung zu jedem Arretiervorsprung eine zum Einführen eines Arretiervorsprungs in einem Umfangsbereich offene Arretieraussparung vorgesehen ist.

Um dabei die Arretierwirkung in einfacher Weise lösen bzw. auch herstellen zu können, kann vorgesehen sein, dass der wenigstens eine Arretiervorsprung in Richtung einer Vorsprungslängsachse zwischen einer Aufnahmestellung zum Einführen in eine Arretieraussparung und einer Arretierstellung bewegbar ist, wobei in der Arretierstellung ein Arretierabschnitt des Arretiervorsprungs in einen Gegen-Arretierabschnitt der Arretieraussparung derart eingreift, dass der Arretiervorsprung nicht aus der Arretieraussparung herausbewegbar ist.

Ein ungewünschtes Lösen des Arretierzustands kann dadurch verhindert werden, dass der wenigstens eine Arretiervorsprung in seine Arretierstellung vorgespannt ist.

Die Arretierwechselwirkung zwischen dem Führungsbahnelement und dem Schwenkträgerelement kann weiter verbessert werden, wenn am Schwenkträgerelement zwei sich entlang einer gemeinsamen Vorsprungslängsachse erstreckende Arretiervorsprünge vorgesehen sind und wenn am Schwenk/Arretierbereich des Führungsbahnelements zwei jeweils eine Arretieraussparung aufweisende Arretieraussparungsvorsprünge vorgesehen sind.

Gemäß einem weiteren besonders vorteilhaften Aspekt der vorliegenden Erfindung kann vorgesehen sein, dass die Optikträgeranordnung einen am proximalen Ende des Vorrichtungskörpers positionierten oder positionierbaren Führungsbahnbereich umfasst mit einer die Vorrichtungskörperlängsachse ringartig umgebenden ersten Führungsformation, vorzugsweise Führungsnut oder Führungsfläche, in einer Baugruppe von Führungsbahnbereich und Optikträger, vorzugsweise dem Führungsbahnbereich, und mit einer zum Bewegen des Optikträgers auf seiner Bewegungsbahn um die Vorrichtungskörperlängsachse entlang der ersten Führungsformation geführten zweiten Führungsformation, vorzugsweise Führungsvorsprung oder Führungsring, an der anderen Baugruppe von Führungsbahnbereich und Optikträger, vorzugsweise dem Optikträger.

Das Vorsehen einer ersten Führungsformation, z. B. Führungsnut oder Führungsfläche, vorteilhafterweise am Führungsbahnbereich bzw. am Vorrichtungskörper und das Vorsehen einer mit dieser Führungsformation zusammenwirkenden zweiten Führungsformation, z. B. Führungsvorsprung oder Führungsring, vorteilhafterweise am Optikträger ermöglicht einerseits einen kompakten, eine vergleichsweise kurze axiale Baugröße benötigenden Aufbau, gewährleistet andererseits jedoch eine definierte Führung bzw. auch eine definierte Fixierung des Optikträgers am Führungsbahnbereich. Dabei kann der Führungsbahnbereich selbstverständlich in der vorangehend beschriebenen Art und Weise durch eine Schwenkbewegung nahe dem bzw. angrenzend an das proximale Ende des Vorrichtungskörpers positioniert werden. Gleichwohl kann die vorteilhafte Wechselwirkung z. B. zwischen einer Führungsnut und einem in diese eingreifenden Führungsvorsprung auch dann erzielt werden, wenn der Führungsbahnbereich in anderer Art und Weise am Vorrichtungskörper vorgesehen wird, beispielsweise durch axiales Aufschieben oder durch Bereitstellen eines integralen oder am Vorrichtungskörper fest vorgesehenen Bestandteils desselben.

Die Führungsnut ist vorteilhafterweise zum Eingriff des Führungsvorsprungs in Richtung der Vorrichtungskörperlängsachse vom proximalen Endbereich des Vorrichtungskörpers weg offen. Somit kann ein gegenseitiges Stören des an dem Führungsbahnbereich vorgesehenen bzw. getragenen Optikträgers mit anderen Systemkomponenten vermieden werden.

Um ein Herausbewegen des Führungsvorsprungs aus der Führungsnut insbesondere dann, wenn der Optikträger nicht bezüglich des Führungsbahnbereichs fixiert ist und entlang der Bewegungsbahn um die Vorrichtungskörperlängsachse bewegt wird, zu verhindern, wird vorgeschlagen, dass die Führungsnut einen Nuthinterschneidungsbereich aufweist und dass am Führungsvorsprung ein in den Nuthinterschneidungsbereich eingreifender Hinterschneidungseingriffsbereich vorgesehen ist.

Bei einer alternativen Ausgestaltungsart kann die erste Führungsformation eine Führungsfläche umfassen, welche die Vorrichtungskörperlängsachse ringartig, vorzugsweise als geschlossener Ring, umgibt. Die zweite Führungsformation kann einen Führungsring umfassen, wobei der Führungsring eine Gegen-Führungsfläche zur Anlage an der Führungsfläche aufweist. Auch diese Gegen-Führungsfläche erstreckt sich dann, wenn diese in Anlage an der Führungsfläche ist, vorzugsweise im Wesentlichen vollständig um die \/orrichtungskörperlängsachse, so dass zwischen dem Führungsbahnbereich und dem Optikträger eine nicht auf einen vergleichsweise kurzen Umfangsbereich begrenzte Führungswechselwirkung besteht, sondern eine ringartige Führungswechselwirkung erzeugt wird. Aufgrund dieser ringartigen Führungswechselwirkung besteht im Wesentlichen keine Gefahr, dass bei am Optikträger getragenen Optiksystem eine Verkantung erzeugt wird, die eine Bewegung des Optikträgers entlang der Bewegungsbahn beeinträchtigen könnte. Ferner werden die Sterilisierung erschwerende Hinterschneidungen vermieden.

Vorteilhafterweise ist eine Fläche von Führungsfläche und Gegen-Fläche, beispielsweise die Führungsfläche, eine Innenumfangsfläche, während die andere Fläche, also beispielsweise die Gegen-Führungsfläche, eine Außenumfangsfläche sein kann.

Um einerseits eine verbesserte Führungswechselwirkung zwischen den beiden Führungsformationen erlangen zu können, andererseits auch einen definierten axialen Zusammenhalt gewährleisten zu können, wird weiter vorgeschlagen, dass in Zuordnung zu einer Fläche von Führungsfläche und Gegen-Führungsfläche eine in Richtung der Bewegungsbahn sich erstreckende Führungseinsenkung und in Zuordnung zu der anderen Fläche ein in Richtung der Bewegungsbahn erstreckender Führungsvorsprung zum Eingriff in die Führungseinsenkung vorgesehen ist.

Um den Führungsring mit seiner Gegen-Führungsfläche in Führungswechselwirkung mit dem Führungsbahnbereich bzw. der dort vorgesehenen Führungsfläche bringen zu können, wird vorgeschlagen, dass der Führungsring radialelastisch ist. Dadurch wird es beispielsweise möglich, den Führungsring zum Einsetzen in den Führungsbahnbereich radial zu komprimieren, und die Kompressionsbelastung bei in den Führungsbahnbereich eingesetztem Führungsring aufzugeben, so dass dieser aufgrund seiner Radialelastizität wieder in seine Ausgangsform zurückkehrt und dabei mit seiner Gegen-Führungsfläche in Führungsanlage an der Führungsfläche kommt.

Eine weiter verbesserte Führungswirkung für den Optikträger kann dadurch erreicht werden, dass im Bereich des proximalen Endes des Vorrichtungskörpers eine weitere die Vorrichtungskörperlängsachse ringartig umgebende Führungsfläche vorgesehen ist und dass am Führungsring eine weitere Gegen-Führungsfläche zur Anlage an der weiteren Führungsfläche vorgesehen ist.

Zur stabilen Fixierung des Optikträgers bezüglich des Vorrichtungskörpers bzw. des Führungsbahnbereichs wird vorgeschlagen, dass eine Baugruppe von Führungsbahnbereich und Optikträger, vorzugsweise der Optikträger, ein gegen die andere Baugruppe von Führungsbahnbereich und Optikträger, vorzugsweise den Führungsbahnbereich, pressbares Fixierorgan zum Fixieren des Optikträgers vorzugsweise in beliebiger Positionierung entlang der Führungsnut bezüglich des Führungsbahnbereichs umfasst. Da die Relativbewegung zwischen dem Optikträger und dem Führungsbahnbereich durch die erste Führungsformtion und die zweite Führungsformation definiert ist, ist weiter gewährleistet, dass eine beliebige Relativpositionierung des Optikträgers entlang seiner kreisförmigen Bewegungsbahn erhalten werden kann.

Wenn an dem Optikträger ein bezüglich der Vorrichtungskörperlängsachse im Wesentlichen von radial außen gegen einen Widerlagerbereich am Führungsbahnbereich pressbares Fixierorgan vorgesehen ist, kann eine stabile Fixierung des Optikträgers bezüglich des Führungsbahnbereichs erreicht werden, ohne dass eine Beeinträchtigung des Öffnungsquerschnitts des rohrartig ausgebildeten Vorrichtungskörpers und somit der zur Durchführung eines operativen Eingriffs gebildeten Öffnung eintritt.

Dabei ist es weiter vorteilhaft, wenn zum Herstellen und Aufheben der Fixierung das Fixierorgan im Wesentlichen radial bezüglich der Vorrichtungskörperlängsachse bewegbar ist. Somit ist gewährleistet, dass eine Fixierkraftbeaufschlagung im Wesentlichen orthogonal zur jeweiligen lokalen Bewegungsrichtung des Optikträgers erfolgt, was eine gleichermaßen stabile Fixierung gegen Bewegung in beiden Richtungen gewährleistet.

Um die Fixierwirkung herstellen bzw. aufheben zu können, wird vorgeschlagen, dass dem Fixierorgan ein Fixierorganstellantrieb zum Verstellen des Fixierorgans zwischen einer Fixierstellung und einer Freigabestellung zugeordnet ist.

Die zum Bewegen des Fixierorgans in Richtung Fixierstellung bzw. in Richtung Freigabestellung erforderliche Betätigungskraft kann in einfacher, gleichwohl jedoch zuverlässiger Art und Weise dadurch übertragen werden, dass der Fixierorganstellantrieb einen mit dem Fixierorgan bewegbaren ersten Beaufschlagungsbereich und einen den ersten Beaufschlagungsbereich beaufschlagenden und zum Bewegen des Fixierorgans bezüglich des ersten Beaufschlagungsbereichs verlagerbaren zweiten Beaufschlagungsbereich an einem Beaufschlagungsorgan umfasst, wobei wenigstens ein Beaufschlagungsbereich, vorzugsweise jeder Beaufschlagungsbereich, keilartig ausgebildet ist.

Dabei kann zum Erhalt eines kompakten Aufbaus vorgesehen sein, dass der erste Beaufschlagungsbereich mit dem Fixierorgan in einer ersten Verschieberichtung verschiebbar ist und dass der zweite Beaufschlagungsbereich mit dem Beaufschlagungsorgan in einer zu der ersten Verschieberichtung angewinkelten, vorzugsweise dazu im Wesentlichen orthogonal stehenden zweiten Verschieberichtung verschiebbar ist.

Die zur Bewegung des Fixierorgans erforderliche Bewegung des Beaufschlagungsorgans kann in einfacher Weise dadurch erreicht werden, dass dem Beaufschlagungsorgan ein Gewindeantrieb zugeordnet ist. Vermittels eines derartigen Gewindeantriebs kann eine sehr präzise Verstellung des Beaufschlagungsorgans und somit eine gleichermaßen präzise Verstellung des Fixierorgans erreicht werden, wobei vermittels eines derartigen Gewindeantriebs auch die zum Erreichen einer stabilen Fixierung erforderlichen Kräfte bereitgestellt werden können.

Um vermittels der beiden in Beaufschlagungswechselwirkung stehenden Beaufschlagungsbereiche eine Bewegung des Fixierorgans in beiden möglichen Orientierungen, also in Richtung zur Fixierstellung und in Richtung zur Freigabestellung, gewährleisten zu können, wird weiter vorgeschlagen, dass der erste Beaufschlagungsbereich und der zweite Beaufschlagungsbereich in Formschlusseingriff miteinander stehen.

Zum Anbringen eines Optiksystems an dem Optikträger wird vorgeschlagen, dass an dem Optikträger ein Optikfixierbereich zum Fixieren eines Optiksystems an dem Optikträger vorgesehen ist.

Hierbei kann eine stabile, gleichwohl einfach herzustellende und auch einfach zu lösende Fixierung dadurch erreicht werden, dass der Optikfixierbereich ein Schwalbenschwanzinnenprofil oder ein Schwalbenschwanzaußenprofil, vorzugsweise Schwalbenschwanzinnenprofil, sowie ein zum Herstellen und Aufheben der Fixierung des Optiksystems bezüglich des Optikträgers bewegbares Optiksystemfixierorgan am Optikträger umfasst.

Die vorliegende Erfindung wird nachfolgend mit Bezug auf die beiliegenden Figuren detailliert beschrieben. Es zeigt:
- Fig. 1: eine Seitenansicht einer Vorrichtung zum Bereitstellen einer Zugriffsöffnung in einen Körper mit daran getragenem Optiksystem;
- Fig. 2: eine perspektivische Ansicht der Vorrichtung der Fig. 1 mit dem daran getragenen Optiksystem;
- Fig. 3: in perspektivischer Darstellung eine Optikträgeranordnung der Vorrichtung der Fig. 1 mit dem daran getragenen Optiksystem;
- Fig. 4: eine perspektivische Darstellung der Optikträgeranordnung;
- Fig. 5: die Vorrichtung der Fig. 1 mit in einer Einführrelativpositionierung bezüglich einander positioniertem Schwenk/Arretierbereich der Optikträgeranordnung bezüglich eines Schwenkträgerelements;
- Fig. 6: eine Seitenansicht der Vorrichtung der Fig. 1 mit in der Einführrelativpositionierung bezüglich einander positioniertem Schwenk/Arretierbereich und Schwenkträgerelement;
- Fig. 7: eine der Fig. 6 entsprechende Darstellung mit in einer Betriebsstellung anschließend an ein proximales Ende des Vorrichtungskörpers positionierter Optikträgeranordnung;
- Fig. 8: eine Teil-Schnittdarstellung der Optikträgeranordnung, geschnitten längs einer Linie VIII-VIII in Fig. 7;
- Fig.9: eine weitere Schnittdarstellung der Optikträgeranordnung, geschnitten in einer der Zeichenebene der Fig. 7 entsprechenden Schnittebene;
- Fig. 10: eine weitere Teil-Schnittdarstellung der Optikträgeranordnung, geschnitten längs einer Linie parallel zur Linie VIII-VIII in Fig. 7;
- Fig. 11: in vergrößerter Darstellung einen Führungsvorsprung mit einem Hinterschneidungseingriffsbereich;
- Fig. 12: eine Schnittdarstellung eines Optikträgers der Optikträgeranordnung, geschnitten längs einer Linie XII-XII in Fig. 9;
- Fig. 13: vergrößert und im Schnitt dargestellt ein Detail des Optikträgers, geschnitten längs einer Linie XIII-XIII in Fig. 12;
- Fig. 14: in ihren Darstellungen a) und b) ein Optiksystemfixierorgan in einer Freigabestellung bezüglich eines Gegen-Fixierorgans;
- Fig. 15: in ihren Darstellungen a) und b) das Optiksystemfixierorgan in seiner Fixierstellung bezüglich des Gegen-Fixierorgans;
- Fig. 16: die Vorrichtung der Fig. 1 in Blickrichtung XIV in Fig. 1 bei in einer anderen Relativpositionierung des Optikträgers bezüglich des Vorrichtungskörpers;
- Fig. 17: die Vorrichtung der Fig. 1 in Blickrichtung XV in Fig. 1 bei in der Relativpositionierung der Fig. 14 positioniertem Optikträger;
- Fig. 18: die Vorrichtung der Fig. 1 an einem Gelenkstativ getragen;
- Fig. 19: eine perspektivische Ansicht einer alternativen Ausgestaltungsart einer Vorrichtung zum Bereitstellen einer Zugriffsöffnung in einen Körper mit daran getragenem Optiksystem;
- Fig. 20: eine Ansicht der Vorrichtung der Fig. 19 in Blickrichtung XX in Fig. 19;
- Fig. 21: eine Seitenansicht der Vorrichtung der Fig. 19;
- Fig. 22: eine Teil-Schnittansicht der Vorrichtung der Fig. 19, geschnitten längs einer Linie XXII-XXII in Fig. 21;
- Fig. 23: eine weitere Seitenansicht der Vorrichtung der Fig. 19 mit verschwenkter Optikträgeranordnung;
- Fig. 24: die Optikträgeranordnung der Vorrichtung der Fig. 19 in Explosionsdarstellung;
- Fig. 25: eine perspektivische Ansicht der teilweise geschnitten dargestellten Optikträgeranordnung der Fig. 19;
- Fig. 26: eine Axialansicht der Optikträgeranordnung der Fig. 19 in Blickrichtung XXVI-XXVI in Fig. 25;
- Fig. 27: eine Schnittdarstellung eines Führungsrings der Optikträgeranordnung, geschnitten längs einer Linie XXVII-XXVII in Fig. 26;
- Fig. 28: ein Führungsbahnelement der Optikträgeranordnung in perspektivischer Ansicht;
- Fig. 29: einen Optikträger der Optikträgeranordnung mit daran vorgesehenem Führungsring.

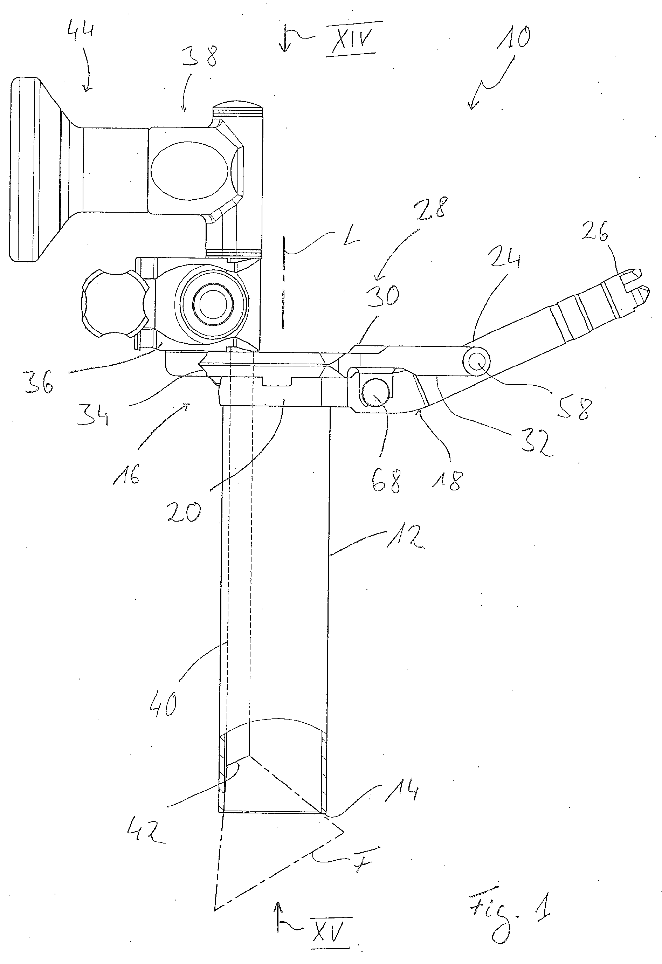

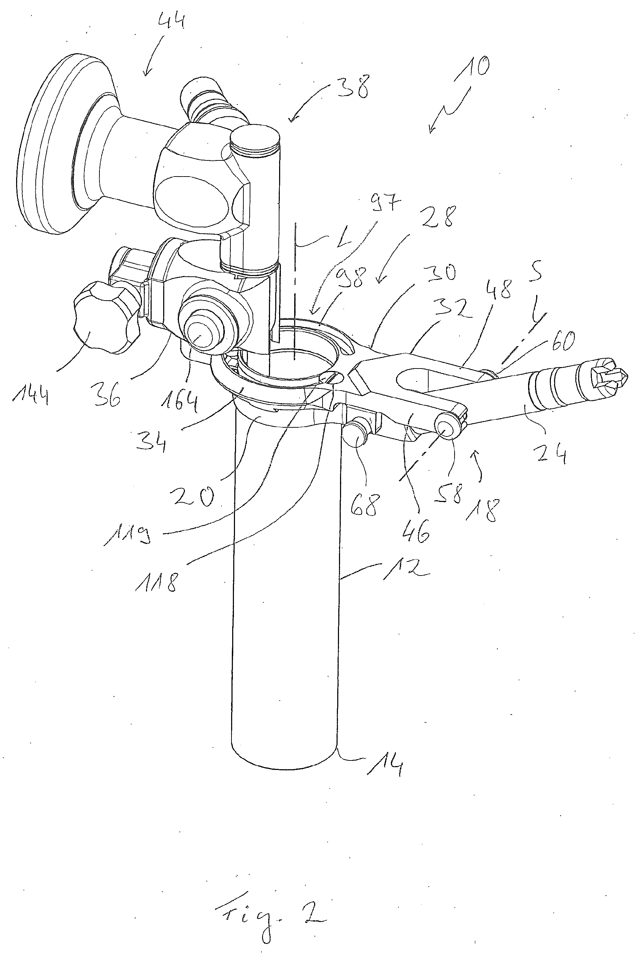

Die Fig. 1 und 2 zeigen eine Vorrichtung 10 zum Bereitstellen einer Zugriffsöffnung in einen Körper bei der Durchführung eines perkutanen operativen Eingriffs an einer Wirbelsäule, beispielsweise im Lendenwirbelbereich oder im Halswirbelbereich. Die Vorrichtung 10 umfasst einen allgemein auch als Trokar bezeichneten, rohrartig ausgebildeten und entlang einer Vorrichtungskörperlängsachse L sich erstreckenden Vorrichtungskörper 12. Dabei ist der Vorrichtungskörper 12 vorteilhafterweise von zylindrischer und kreisrunder Gestalt. Mit einem distalen Ende 14 wird bei Durchführung eines operativen Eingriffs der Vorrichtungskörper 12 in einem zu behandelnden Körper, beispielsweise menschlichen Körper, positioniert. Ein proximales Ende 16 liegt dabei außerhalb des behandelten Körpers, jedoch vergleichsweise nahe an der Haut bzw. Körperoberfläche.

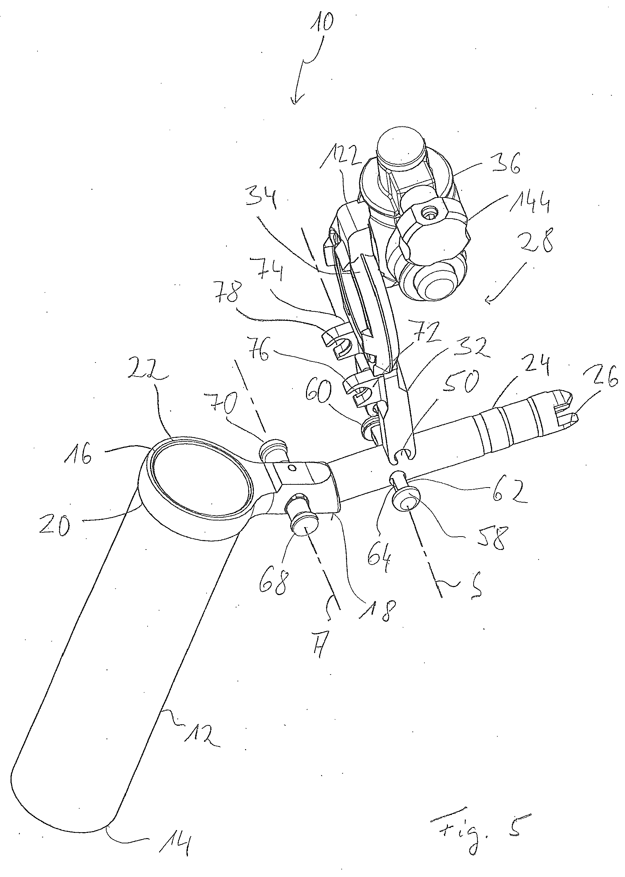

Am Vorrichtungskörper 12 ist nahe dem proximalen Ende 16 ein allgemein mit 18 bezeichnetes Schwenkträgerelement vorgesehen. Dieses umfasst einen den Vorrichtungskörper 12 vorteilhafterweise ringartig umgebenden und mit diesem materialschlüssig, beispielsweise durch Verschweißung oder Verklebung, fest verbundenen Befestigungsbereich 20. Wie dies insbesondere in der Fig. 5 zu erkennen ist, ist der Befestigungsbereich 20 bezüglich des Vorrichtungskörpers 12 derart positioniert, dass er in Richtung der Vorrichtungskörperlängsachse L im Wesentlichen bündig mit dem proximalen Ende 16 bzw. einer dort vorgesehenen Stirnfläche 22 des Vorrichtungskörpers 12 abschließt. Somit ist das Schwenkträgerelement 18 möglichst nahe am proximalen Ende 16 des Vorrichtungskörpers 12 positoniert. Lediglich der von dem Befestigungsbereich 20 überdeckte Längenabschnitt des Vorrichtungskörpers 12 kann somit nicht in einen zu behandelnden Körper eingeführt werden. Wie dies in den Figuren erkennbar ist, ist dieser Längenabschnitt jedoch vergleichsweise kurz. Damit rücken alle nachfolgend noch erläuterten Systembereiche sehr nahe an die Oberfläche des zu behandelnden Körpers heran.

Das Schwenkträgerelement 18 umfasst ferner einen von dem Befestigungsbereich 20 beispielsweise teilweise abgewinkelt sich erstreckenden Trägerbereich 24. Dieser Trägerbereich 24 kann an seinem vom Vorrichtungskörper 12 entfernt liegenden freien Ende 26 zur festen Kopplung mit einem Gelenkstativ ausgebildet sein, so dass die gesamte Vorrichtung 10 während der Durchführung eines operativen Eingriffs stabil gehaltert werden kann. Ferner ist am Trägerbereich 18 eine Optikträgeranordnung 28 um eine zur Zeichenebene der Fig. 1 orthogonal stehende Schwenkachse S schwenkbar.

Die Optikträgeranordnung 28 umfasst ein Führungsbahnelement 30 mit einem mit dem Trägerbereich 24 des Schwenkträgerelements 18 schwenkbar zu koppelnden Schwenk/Arretierbereich 32 und einem in der in Fig. 1 und 2 erkennbaren Betriebsstellung des Führungsbahnelements 30 anschließend bzw. axial angrenzend an das proximale Ende 16 des Vorrichtungskörpers 12 positionierten Führungsbahnbereich 34.

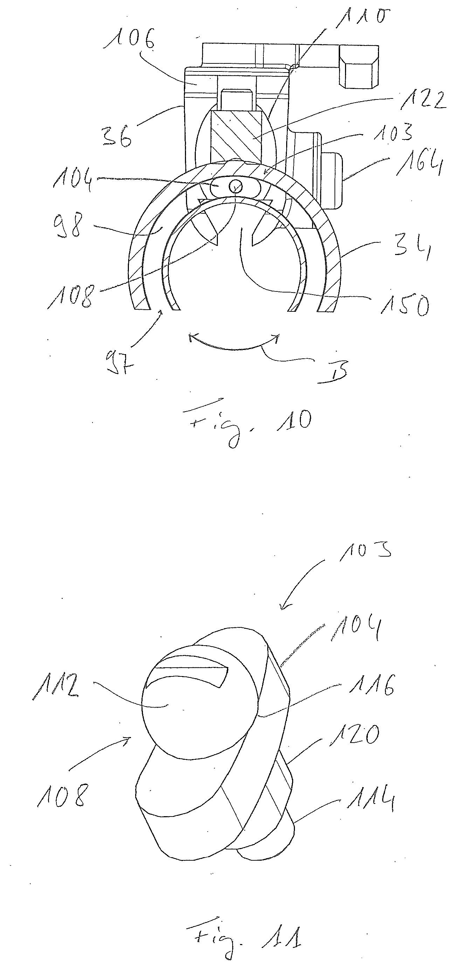

An dem Führungsbahnbereich 34 ist ein Optikträger 36 derart getragen, dass dieser einerseits entlang einer die Vorrichtungskörperlängsachse L ringartig umgebenden Bewegungsbahn B (siehe Fig. 10) bewegbar ist, jedoch an beliebiger Positionierung entlang dieser Bewegungsbahn B bezüglich des Führungsbahnbereichs 34 fixierbar ist.

Am Optikträger 36 kann dann, wenn das Führungsbahnelement 30 in seiner Betriebsstellung positioniert ist, ein allgemein mit 38 bezeichnetes Optiksystem fixiert werden. Das Optiksystem 38 umfasst einen im Inneren des Vorrichtungskörpers 12 sich erstreckenden, stabartigen Optikbereich 40, der an der Innenoberfläche des Vorrichtungskörpers 12, also exzentrisch zur Vorrichtungskörperlängsachse L verläuft und beispielsweise im Inneren des Vorrichtungskörpers 12 jedoch nahe dem distalen Ende 14 liegend eine Optiköffnung 42 aufweist. Diese Optiköffnung 42 bzw. die im Optikbereich 40 vorgesehenen optischen Elemente definieren ein Sichtfeld F durch das distale Ende 14 des Vorrichtungskörpers 12 hindurch, das durch Bewegung des Optikträgers 36 und damit des Optiksystems 38 entlang der Bewegungsbahn B variiert werden kann, um den im Verlaufe eines operativen Eingriffs zu behandelnden Bereich umfassend optisch erkennen zu können.

Das Optiksystem 38 umfasst ferner einen Bildgebungsbereich 44, in welchem beispielsweise durch visuelle Beobachtung mittels eines Auges, gleichermaßen aber auch durch elektronische Bilderfassung, der durch das Sichtfeld F beobachtete Bereich des behandelten Körpers erkennbar wird bzw. dargestellt werden kann, bzw. ein Anschluss an einen Bildschirm hergestellt werden kann.

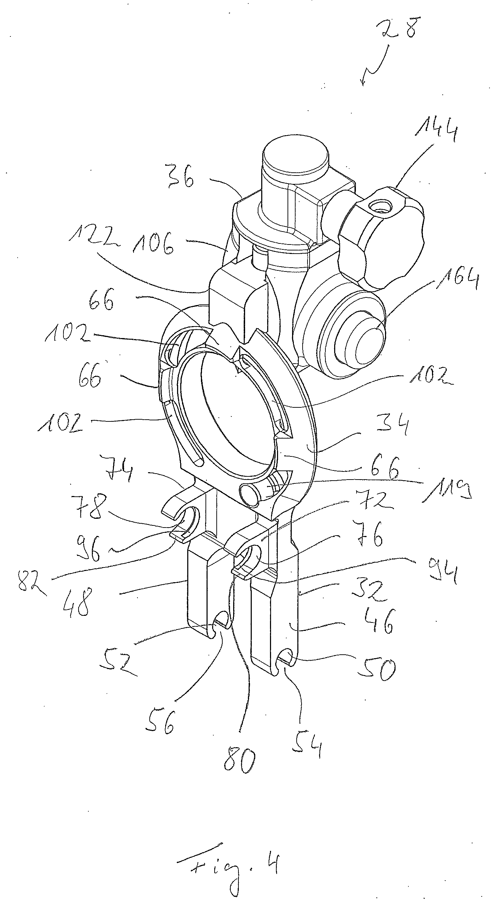

Die in Fig. 4 dargestellte Optikträgeranordnung 28 ist im Schwenk/Arretierbereich 32 des Führungsbahnelements 30 gabelartig ausgebildet mit zwei zueinander vorzugsweise im Wesentlichen parallel sich erstreckenden Gabelendbereichen 46, 48. In jedem dieser Gabelendbereiche 46, 48 ist eine Schwenkaussparung 50, 52 mit beispielsweise im Wesentlichen kreisrunder Profilierung vorgesehen. In einem Umfangsbereich dieser beispielsweise kreisrunden Profilierung ist in jedem Gabelendbereich 46, 48 eine Öffnung 54, 56 gebildet, so dass jede Schwenkaussparung 50, 52 in diesem Umfangsbereich beispielsweise in einer Längserstreckungsrichtung der Gabelendbereiche 46, 48 offen ist.

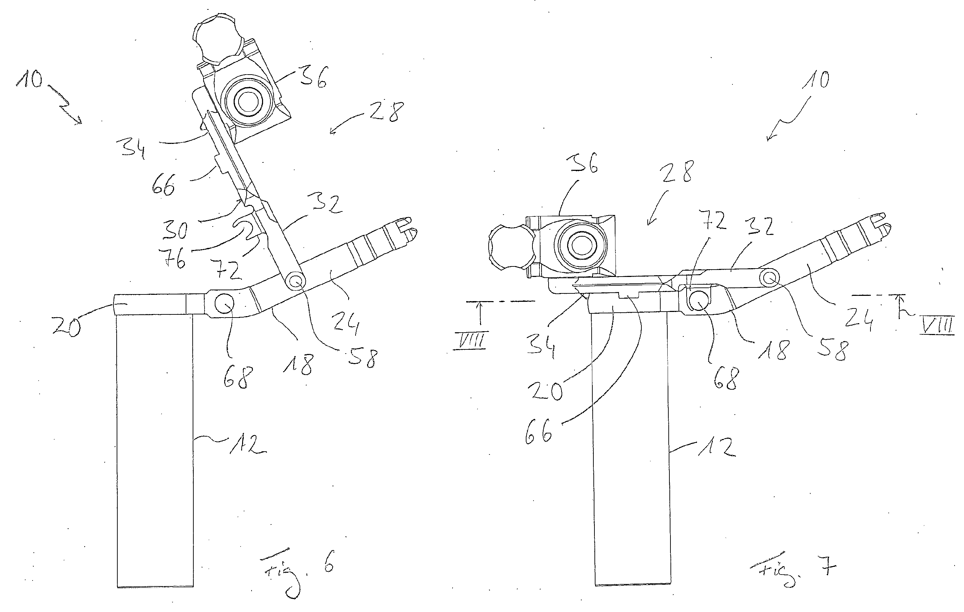

Am Trägerbereich 24 des Schwenkträgerelements 18 sind im Wesentlichen parallel zueinander und eine gemeinsame Schwenkachse S definierend zwei beispielsweise pilzartig ausgebildete Schwenkvorsprünge 58, 60 vorgesehen. Diese Schwenkvorsprünge 58, 60 sind derart dimensioniert und am Trägerbereich 24 positioniert, dass sie bei in einer Einführrelativpositionierung des Führungsbahnelements 30 bezüglich des Schwenkträgerelements 18 in eine jeweils zugeordnete Schwenkaussparung 50, 52 eingeführt werden können. Dazu sind die Führungsvorsprünge 58, 60 an jeweils zwei einander diametral gegenüberliegenden Bereichen 62, 64 abgeflacht, während sie ansonsten eine im Wesentlichen kreisrunde Außenumfangskontur aufweisen können, welche an die kreisrunde Innenkontur der Schwenkaussparungen 50, 52 angepasst sein kann. Somit ist mit den beiden einander diametral gegenüberliegenden, abgeflachten Bereichen 62, 64 ein Umfangsbereich eines jeweiligen Schwenkvorsprungs 58, 60 gebildet, in welchem dieser eine geringere Querschnittsabmessung, also einen geringeren Durchmesser, aufweist, als in einem dazu beispielsweise um 90° versetzt liegenden Umfangsbereich. In der in den Fig. 5 und 6 erkennbaren Einführrelativpositionierung ist dieser Umfangsbereich mit geringerer Querschnittsabmessung so positioniert, dass die Schwenkvorsprünge 58, 60 durch die Öffnungen 54, 56 in den Gabelendbereichen 46, 48 hindurchgeführt werden können. Dabei zeigt die Fig. 5 den Zustand vor dem Aufschieben der Gabelendbereiche 46, 48 auf die Schwenkvorsprünge 58, 60, während die Fig. 6 den Zustand bei in den Schwenkaussparungen 50, 52 bereits aufgenommenen Schwenkvorsprüngen 58, 60, jedoch noch nicht bezüglich einander verschwenktem Führungsbahnelement 30 und Schwenkträgerelement 18 zeigt.

Ausgehend von der in Fig. 6 dargestellten Einführrelativpositionierung kann das Führungsbahnelement 30 in der Darstellung der Fig. 6 im Gegenuhrzeigersinn um die Schwenkachse S verschwenkt werden, so dass der Führungsbahnbereich 34 sich dem proximalen Ende 16 des Vorrichtungskörpers 12 annähert. In der in Fig. 7 dargestellten Betriebsstellung des Führungsbahnelements 30 liegt der Führungsbahnbereich 34 am proximalen Ende 16 bzw. der Stirnfläche 22 des Vorrichtungskörpers 12 oder/und an dem ringartigen Befestigungsbereich 20 des Schwenkträgerelements 18 an. Da bereits nach geringfügiger Verschwenkung des Führungsbahnelements 30 ausgehend von der in Fig. 6 dargestellten Einführrelativpositionierung die abgeflachten Bereiche 62, 64 der Schwenkvorsprünge 58, 60 nicht mehr mit den Öffnungen 54, 56 der Schwenkaussparungen 50, 52 ausgerichtet sind, kann auch im Verlaufe der Schwenkbewegung in Richtung zur Betriebsstellung ein ungewolltes Loslösen des Führungsbahnelements 30 vom Schwenkträgerelement 18 nicht auftreten. Da ferner die Schwenkvorsprünge 58, 60 mit ihren Bereichen größerer Umfangsabmessung, also größeren Durchmessers, auf die Schwenkaussparungen 50, 52 abgestimmt sind, ist eine Relativbewegung des Führungsbahnelements 30 bezüglich des Schwenkträgerelements 18, ungeachtet der selbstverständlich möglichen Schwenkbewegung, auf ein durch ein unvermeidbares Bewegungsspiel zwischen den Gabelendbereichen 46, 48 und den Schwenkvorsprüngen 58, 60 definiertes Ausmaß beschränkt. Dies führt dazu, dass in der Betriebsstellung der Führungsbahnbereich 34 eine exakte Positionierung bezüglich des Vorrichtungskörpers 12 einnehmen wird. Dies kann weiter dadurch unterstützt werden, dass am Führungsbahnbereich 34 an mehreren Umfangsbereichen oder in ringartiger Ausgestaltung Positioniervorsprünge 66 vorgesehen sind, die in der Betriebsstellung den Vorrichtungskörper 12 im Bereich seines proximalen Endes 16 bzw. den den Vorrichtungskörper 12 in diesem Bereich ringartig umgebenden Befestigungsbereich 20 des Schwenkträgerelements 18 radial außen bezüglich der Vorrichtungskörperlängsachse L axial übergreifen und somit eine Relativbewegung des Führungsbahnelements 30 bezüglich des Vorrichtungskörpers 12 zusätzlich verhindern. Es ist darauf hinzuweisen, dass, obgleich in der Betriebsstellung diese Positioniervorsprünge 66 den Vorrichtungskörper 12 geringfügig axial übergreifen, im Sinne der vorliegenden Erfindung das Führungsbahnelement 30 insbesondere mit seinem Führungsbahnbereich 34 axial anschließend an das proximale Ende des Vorrichtungskörpers 12 und somit im Bereich dieses proximalen Endes 16 positioniert ist. Ein derartiger insbesondere über die axiale Ausdehnung des Befestigungsbereichs 20 bzw. allgemein des zur Festlegung des Schwenkträgerelements 18 am Vorrichtungskörper 12 erforderlichen axialen Längenbereichs des Vorrichtungskörpers 12 bestehender Übergriff bedeutet gleichwohl, dass im Sinne der vorliegenden Erfindung das Führungsbahnelement 34 anschließend, also axial angrenzend an das proximale Ende 16 des Vorrichtungskörpers 12 positioniert ist. Selbst ein Übergreifen des Vorrichtungskörpers 12 in Richtung der Vorrichtungskörperlängsachse L geringfügig auch über den zur Befestigung des Schwenkträgerelements 18 erforderlichen axialen Bereich hinaus steht dem nicht entgegen.

Um eine Arretierung des Führungsbahnelements 30 in der Betriebsstellung zu erreichen, sind am Schwenkträgerelement 18 zwei vom Trägerbereich 24 desselben sich im Wesentlichen entgegengesetzt, jedoch entlang einer gemeinsamen Vorsprungslängsachse A sich erstreckende, beispielsweise im Wesentlichen pilzartig ausgebildete Arretiervorsprünge 68, 70 vorgesehen. In Zuordnung zu jedem Arretiervorsprung 68, 70 ist am Schwenk/Arretierbereich 32 des Führungsbahnelements 30 ein beispielsweise im Wesentlichen orthogonal vom Schwenk/Arretierbereich 32 sich erstreckender Arretieraussparungsvorsprung 72, 74 vorgesehen. In jedem Arretieraussparungsvorsprung 72, 74 ist eine Arretieraussparung 76, 78 beispielsweise mit im Wesentlichen kreisrunder Profilierung ausgebildet. In ihren vom Schwenk/Arretierbereich 32 weg orientierten Endbereichen weisen die Arretieraussparungsvorsprünge 72, 74 Öffnungen 80, 82 auf, so dass die Arretieraussparungen 76, 78 in einem einer jeweiligen Öffnung 80, 82 entsprechenden Umfangsbereich offen sind.

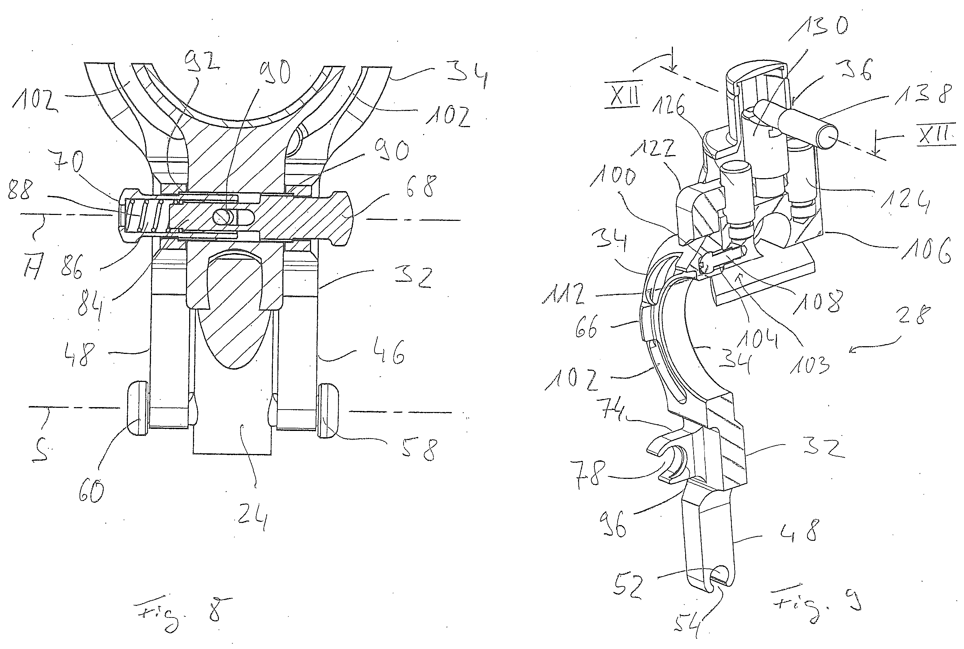

Die Fig. 8 zeigt, dass die Arretiervorsprünge 68, 70 am Schwenk/Arretierbereich 32 in Richtung der Vorsprungslängsachse A verschiebbar getragen sind. Dazu ist der Arretiervorsprung 68 mit einem Eingriffsende 84 ausgebildet, während der Arretiervorsprung 70 mit einer Eingriffsöffnung 86 ausgebildet ist, in welcher das Eingriffsende 84 positioniert ist. Durch eine beispielsweise als Schraubendruckfeder ausgebildete Vorspannvorrichtung 88 sind die beiden Arretiervorsprünge 68, 70 in Richtung voneinander weg und somit in Richtung zu einer Arretierstellung vorgespannt. Durch eine Langloch/Bolzen-Anordnung 90 ist das Ausmaß der Verlagerung der beiden Arretiervorsprünge 68, 70 voneinander weg und z. B. auch in Richtung aufeinander zu begrenzt.

An jedem der Arretiervorsprünge 68, 70 ist ein beispielsweise durch eine stufenartige Erweiterung der Querschnittsabmessung desselben gebildeter Arretierabschnitt 90 bzw. 92 gebildet. In Zuordnung dazu ist an jedem Arretieraussparungsvorsprung 72, 74 ein Gegen-Arretierabschnitt 94, 96 vorgesehen, der beispielsweise durch eine stufenartige Erweiterung der Innenquerschnittsabmessung der Arretieraussparungen 76, 78 an deren einander zugewandt liegenden Seiten gebildet sein kann.

Zum Herstellen des Arretierzustands im Verlaufe der Schwenkbewegung des Führungsbahnelements 30 in Richtung zu seiner Betriebsstellung können beispielsweise durch Beaufschlagung vermittels Daumen und Zeigefinger die beiden Arretiervorsprünge 68, 70 in Richtung aufeinander zu belastet werden, so dass sie ausgehend von der in Fig. 8 erkennbaren Arretierstellung einander angenähert werden. Im Verlaufe der Schwenkbewegung kommen die Arretieraussparungsvorsprünge 72, 74 mit ihren Öffnungen 80, 82 in eine Positionierung, in welcher sie mit den Bereichen geringerer Querschnittsabmessung der Arretiervorsprünge 68, 70 ausgerichtet sind, so dass diese im Wesentlichen ungehindert in die Arretieraussparungen 76, 78 eintreten können. Hierzu könnte auch vorgesehen sein, dass durch Bereitstellen entsprechender Abweisschrägen an den Arretieraussparungsvorsprüngen 72, 74 die Arretiervorsprünge 68, 70 entgegen der Vorspannwirkung der Vorspannvorrichtung 88 gegeneinander gepresst werden, so dass die Arretieraussparungsvorsprünge 72, 74 über die Bereiche geringerer Querschnittsabmessung der Arretiervorsprünge 68, 70 geschoben werden können. Ist das Führungsbahnelement 30 in seiner Betriebsstellung, sind die Arretiervorsprünge 68, 70 in maximalem Ausmaß in die Arretieraussparungen 76, 78 eingeführt. Bei Wegfall der Beaufschlagungswirkung der Arretiervorsprünge 68, 70 können diese, der Vorspannwirkung der Vorspannvorrichtung 88 folgend, in ihre Arretierstellung gelangen, in welcher die Arretierabschnitte 90, 92 in die Gegen-Arretierabschnitte 94, 96 eintreten, so dass ein Herausbewegen der Arretiervorsprünge 68, 70 aus den Arretieraussparungen 76, 78 nicht mehr möglich ist. Nur durch Pressen der Arretiervorsprünge 68, 70 aufeinander zu und Herausbewegen der Arretierabschnitte 90, 92 aus den Gegen-Arretierabschnitten 94, 96 der Arretieraussparungen 76, 78 kann die Arretierwechselwirkung aufgehoben werden und das Führungsbahnelement 30 aus seiner Betriebsstellung heraus beispielsweise in Richtung zur Einführrelativpositionierung verschwenkt werden.

Wie dies in den Fig. 1, 6 und 7 deutlich sichtbar ist, liegen die Schwenkvorsprünge 58, 60 einerseits und die Arretiervorsprünge 68, 70 andererseits aufgrund der angewinkelten Ausgestaltung des Schwenkträgerelements 18 in seinem Trägerbereich 24 in zueinander unterschiedlichen axialen Bereichen bezüglich der Vorrichtungskörperlängsachse L. Dies ermöglicht es, dass in der Betriebsstellung das mit im Allgemeinen ungekrümmter Konfiguration ausgebildete Führungsbahnelement 30 im Wesentlichen orthogonal zur Vorrichtungskörperlängsachse L positioniert ist und somit mit seinem Führungsbahnbereich 34 plan und ohne Neigung am proximalen Ende 16 des Vorrichtungskörpers 12 positioniert werden kann. Selbstverständlich wäre hier grundsätzlich auch eine angewinkelte Positionierung, beispielsweise bei entsprechend abgeschrägter Ausgestaltung des proximalen Endes 16, möglich.

Entsprechend der im Wesentlichen rohrartigen Konfiguration des Vorrichtungskörpers 12 ist der Führungsbahnbereich 34 des Führungsbahnelements 30 im Wesentlichen ringartig gestaltet. In diesem Führungsbahnbereich 34 ist als eine erste Führungsformation 97 eine in Richtung vom proximalen Ende 16 des Vorrichtungskörpers 12 weg offene Führungsnut 98 gebildet. Diese erstreckt sich über einen Umfangsbereich von nahezu 360° um die Vorrichtungskörperlängsachse L und ist lediglich dort unterbrochen, wo der Führungsbahnbereich 34 an den Schwenk/Arretierbereich 32 anschließt.

Wie die Fig. 9 dies zeigt, ist die Führungsnut 98 in ihrem vom axial offenen Ende derselben entfernt liegenden Nutgrundbereich mit einem entlang der Führungsbahn 98 sich erstreckenden Nuthinterschneidungsbereich 100 ausgebildet. Ferner kann an mehreren Umfangspositionierungen, insbesondere im Bereich zwischen den in Umfangsrichtung benachbarten Positioniervorsprüngen 66, der Führungsbahnbereich 34 langlochartige Öffnungen 102 aufweisen, so dass die Führungsnut 98 in einigen Umfangsbereichen axial durchgehend offen ist.

Am Optikträger 36 ist als eine zweite Führungsformation 103 ein in die Führungsnut 98 eingreifend positionierter Führungsvorsprung 104 vorgesehen. Dieser in Fig. 11 vergrößert dargestellte Führungsvorsprung 104 ist in Umfangsrichtung der Führungsnut 98 langgestreckt und angepasst an die kreisartig gekrümmte Kontur der Führungsnut 98 gekrümmt. Der Führungsvorsprung 104 ist so dimensioniert, dass er insbesondere in radialer Richtung bezüglich der Vorrichtungskörperlängsachse L im Wesentlichen bewegungsspielfrei in der Führungsnut 98 aufgenommen ist.

Der Führungsvorsprung 104 könnte mit einem Optikträgerkörper 106 des Optikträgers 36 integral ausgebildet sein. Im dargestellten Beispiel ist der Führungsvorsprung 104 als separates Bauteil ausgebildet und durch einen Schraubbolzen 108 an einer dem Führungsbahnbereich 34 zugewandten Seite 110 des Optikträgerkörpers 106 festgelegt. Dazu kann der Schraubbolzen 108 einerseits den Führungsvorsprung 104 mit seinem Bolzenkopf 112 übergreifen und andererseits mit einem Gewindeabschnitt 114 in eine Gewindeöffnung des Optikträgerkörpers 106 eingeschraubt und dort beispielsweise durch Verklebung gegen ungewolltes Loslösen festgelegt werden.

Man erkennt in Fig. 11, dass der Bolzenkopf 112 eine größere Querabmessung aufweist, als der Führungsvorsprung 104. Dies bedeutet, dass der Bolzenkopf 112 in radialer Richtung über den Führungsvorsprung 104 hervorsteht und, wie die Fig. 9 dies zeigt, somit in den Nuthinterschneidungsbereich 100 eingreifend positioniert werden kann. Der Bolzenkopf 112 bildet somit mit wenigstens einem radial über den Führungsvorsprung 104 vorspringenden Bereich einen Hinterschneidungseingriffsbereich 116.

Zur Montage des Optikträgers 34 an dem Führungsbahnelement 30 kann so vorgegangen werden, dass zunächst der Bolzen 108 mit seinem Bolzenkopf 112 durch eine im Führungsbahnelement 30 an einem Umfangsendbereich der Führungsnut 98 gebildete, bezüglich der Radialerstreckung der Führungsnut 98 erweiterte Montageöffnung 118 hindurchgeführt und in Umfangsrichtung dann in den Umfangsbereich einer der Öffnungen 102 bewegt wird. Die Montageöffnung 118 wird dann durch ein Abschlusselement, beispielsweise eine Abschlussschraube 119, verschlossen, so dass ein Herauslösen des Bolzens 108 aus der Führungsnut 98 nicht mehr möglich ist. Daraufhin kann der Führungsvorsprung 104 axial in die Führungsnut 98 eingeführt bzw. über den Schaft des Bolzens 108 geführt werden. In einem nächsten Schritt wird der Optikträger 36 über dem Führungsvorsprung 104 positioniert und axial auf diesen zu bewegt. Um eine definierte Relativpositionierung zwischen dem Optikträger 36 und dem Führungsvorsprung 104 zu gewährleisten, kann am Führungsvorsprung 104 ein Ausrichtvorsprung 120 mit nicht rotationssymmetrischer Außenumfangskontur ausgebildet sein, der in eine komplementär geformte Aussparung an der Seite 110 des Optikträgerkörpers 106 eingepasst werden kann und somit eine gegen Relativverdrehung gesicherte Ausrichtung des Führungsvorsprungs 104 bezüglich des Optikträgerkörpers 106 gewährleistet. Darauf folgend kann mit einem Schraubwerkzeug durch eine der Öffnungen 102 hindurch gegriffen und auf den Bolzenkopf 112 eingewirkt werden, um den Gewindeabschnitt 114 des Bolzens 108 in die zugeordnete Innengewindeöffnung im Optikträgerkörper 106 einzuschrauben. Es wird dann ein Zustand erhalten, in welchem aufgrund einer passgenauen Positionierung des Führungsvorsprungs 104 und auch des Hinterschneidungseingriffsbereichs 116 in der Führungsnut 98 eine präzise Positionierung und auch Führung des Optikträgers 36 entlang der Führungsnut 98 und somit entlang der kreisartigen Bewegungsbahn B ermöglicht ist.

Zur Fixierung des Optikträgers 36 bezüglich des Führungsbahnbereichs 34 ist am Optikträger 36 ein Fixierorgan 122 vorgesehen. Dieses umgreift den Führungsbahnbereich 34 bezüglich der Vorrichtungskörperlängsachse L von radial außen. Um eine insbesondere auch in axialer Richtung stabile Fixierung zu erlangen, ist der Führungsbahnbereich 34 an seinem Außenumfangsbereich beispielsweise mit konvex V-artiger Profilierung ausgebildet und wird vom Fixierorgan 122 mit einer entsprechenden konkav V-artigen Profilierung übergriffen. Das Arretierorgan 122 ist am Optikträgerkörper 106 im Wesentlichen in radialer Richtung bezüglich der Vorrichtungskörperlängsachse L bewegbar getragen. Um eine definierte Bewegungsführung zu erlangen, sind im Inneren des Optikträgerkörpers 106, welcher aus mehreren Teilen aufgebaut sein kann, zwei Führungsbolzen 124, 126 vorgesehen, welche in entsprechende Führungsöffnungen eines im Inneren des Optikträgerkörpers 106 sich erstreckenden Abschnitts 128 des Fixierorgans 122 eingreifen. Somit ist das Fixierorgan 122 gegen Verkippen gesichert und in radialer Richtung bewegbar.

Zwischen den beiden Führungsbolzen 124, 126 ist ein Beaufschlagungsbolzen 130 mit dem Fixierorgan 122 fest verbunden. Dieser weist in seinem vom Fixierorgan 122 entfernt liegenden Endbereich einen mit einer bzw. mehreren Keilflächen 132 versehenen und somit keilartig ausgebildeten ersten Beaufschlagungsbereich 134 auf. Durch die Führungsbolzen 124, 126 ist das Fixierorgan 122 und damit der mit diesem fest verbundene Beaufschlagungsbolzen 130 zur Bewegung in einer ersten Verschieberichtung R₁ bezüglich des Optikträgerkörpers 106 geführt.

In einem seitlichen Ansatz 136 am Optikträger 36 ist ein stift- bzw. bolzenartiges Beaufschlagungsorgan 138 zur Bewegung in einer zweiten Verschieberichtung R₂ geführt. Diese zweite Verschieberichtung R₂ ist im Wesentlichen orthogonal zur ersten Verschieberichtung R₁. Das Beaufschlagungsorgan 138 weist einen zweiten Beaufschlagungsbereich 140 auf, mit welchem das Beaufschlagungsorgan 138 in Beaufschlagungswechselwirkung mit dem ersten Beaufschlagungsbereich 134 am Beaufschlagungsbolzen 130 steht. Der zweite Beaufschlagungsbereich 140 ist im Wesentlichen bereitgestellt durch einen Längsendbereich des Beaufschlagungsorgans 136, wobei auch hier eine oder merhrere Keilflächen 141 vorgesehen sein können, welche mit den Keilflächen 132 des ersten Beaufschlagungsbereichs 134 in Beaufschlagungswechselwirkung sind. Grundsätzlich ist jedoch zum Erhalt eines Keilschiebergetriebes das Ausgestalten eines Beaufschlagungsbereichs, hier z. B. des ersten Beaufschlagungsbereichs 134, mit keilartiger Konfiguration ausreichend.

Die Bewegung des Beaufschlagungsorgans 138 in der zweiten Verschieberichtung R₂ wird durch einen allgemein mit 142 bezeichneten Gewindeantrieb erreicht. Dieser umfasst ein beispielsweise durch Handbetätigung drehbares Betätigungsrad 144, dessen Drehung über miteinander in Kämmeingriff stehende Gewindebereiche in eine Axialverschiebung des Beaufschlagungsorgans 138 umgesetzt werden kann. Mit dem Betätigungsrad 144 ist eine am Optikträgerkörper 106 drehbar getragene Innengewindehülse 147 drehfest verbunden. Ein am Beaufschlagungsorgan 138 vorgesehenes Außengewinde steht mit dem Innengewinde dieser Innengewindehülse 147 in Eingriff. Da durch einen zwischen dem Beaufschlagungsorgan 138 und dem Beaufschlagungsbolzen 130 bestehenden Formschluss das Beaufschlagungsorgan 138 sich nicht um seine Längsachse drehen kann, wird es bei Drehung der axial grundsätzlich am Optikträgerkörper 106 feststehend gehaltenen Gewindehülse 147 zwangsweise in seiner Längsrichtung verschoben, wodurch, je nach Orientierung dieser Verschiebung, durch den Formschlusseingriff der Beaufschlagungsbolzen 130 in die eine oder die andere Orientierung der ersten Verschieberichtung R₁ bewegt wird.

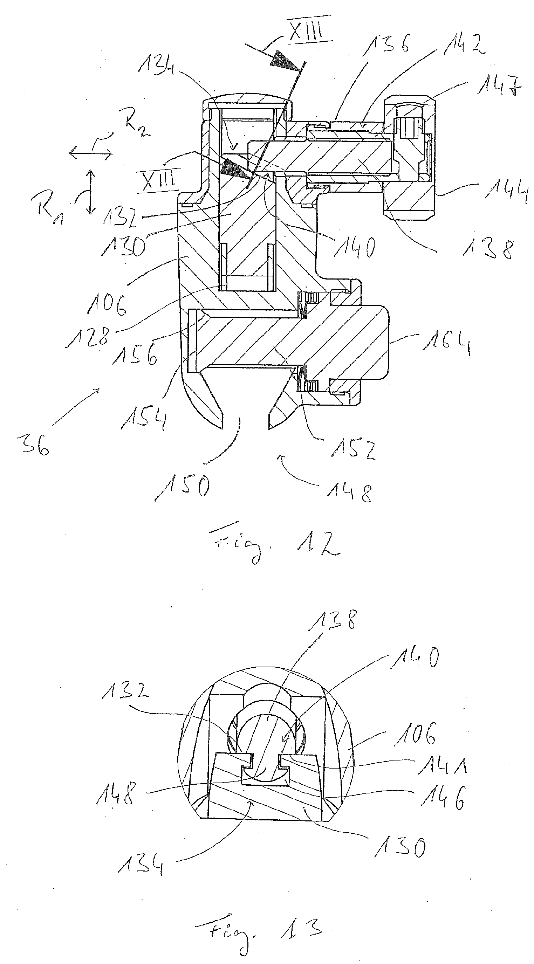

Das Beaufschlagungsorgan 138 und der Beaufschlagungsbolzen 130 stehen im Bereich der beiden Beaufschlagungsbereiche 134, 140 miteinander in Formschlusseingriff. Hierzu kann beispielsweise eine mit Hinterschneidung gebildete Nut 146 am Beaufschlagungsbolzen 130 vorgesehen sein, in welcher ein entsprechender Eingriffsbereich 148 am Beaufschlagungsorgan 138 geführt ist. Somit kann eine Kraftübertragung zwischen den beiden Beaufschlagungsbereichen 134, 140 in beiden Orientierungen der ersten Verschieberichtung R₁ erreicht werden, so dass eine Verschiebung des Beaufschlagungsorgans 138 in der Darstellung der Fig. 12 nach links zu einer Verschiebung des Beaufschlagungsbolzens 130 nach unten und mithin zum Bewegen des Fixierorgans 122 in seine den Führungsbahnbereich 34 beaufschlagende Fixierstellung führt. Wird das Beaufschlagungsorgan 138 in der Darstellung der Fig. 12 nach rechts bewegt, so zieht es den Beaufschalgungsbolzen 130 nach oben, d. h. vom Führungsbahnbereich 34 weg. Dieser Bewegung folgt das Fixierorgan 122, so dass die Fixierwechselwirkung mit dem Führungsbahnbereich 34 aufgehoben wird und der Optikträger 36 zur Bewegung entlang der Führungsnut 98 freigegeben ist.

Es ist darauf hinzuweisen, dass hier grundsätzlich auch eine Anordnung denkbar ist, bei welcher das Beaufschlagungsorgan 138 den Beaufschlagungsbolzen 130 und damit das Fixierorgan 122 nur in Richtung Fixierstellung, also in der Fig. 12 nach unten beaufschlagen kann, während die gegenläufige Bewegung des Fixierorgans 122 bzw. des Beaufschlagungsbolzens 130 durch eine zwischen dem Fixierorgan 122 bzw. dem Beaufschlagungsbolzen 130 und dem Optikträgerkörper 106 wirkende Vorspannanordnung, beispielsweise Schraubendruckfeder, erfolgt.

Die Fig. 12 zeigt weiter einen am Optikträger 36 vorgesehenen Optikfixierbereich 148. Dieser umfasst ein Schwalbenschwanzinnenprofil 150 und ein am Optikträger 36 gegen Federvorspannung verschiebbares Optiksystemfixierorgan 152. Das Optiksystemfixierorgan 152 weist in seinem Endbereich 154 einen kegelstumpfartig erweiterten Fixierbereich 156 auf, der bei der in Fig. 12 dargestellten Positionierung des Optiksystemfixierorgans 152 in die Kontur des Schwalbenschwanzinnenprofils 150 eingreift.

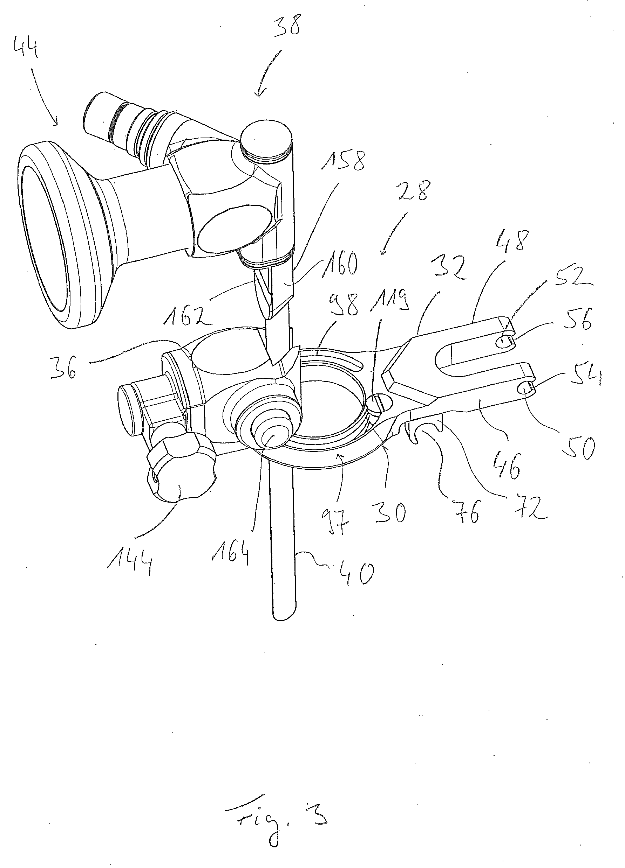

Am Optiksystem 38 ist ein in Fig. 3 erkennbares Gegen-Fixierorgan 158 vorgesehen, welches mit einem zum Schwalbenschwanzinnenprofil 150 komplementären Schwalbenschwanzaußenprofil 160 ausgebildet ist. In Zuordnung zu dem Optiksystemfixierorgan 152 ist in dem Gegen-Fixierorgan 158 eine der Erstreckungsrichtung des Optiksystemfixierorgans 152 entsprechende Einsenkung 162 vorgesehen. Bei vollständig in das Schwalbenschwanzinnenprofil 150 am Optikträger 36 eingeführtem Schwalbenschwanzaußenprofil 160 liegt die Einsenkung 162 dem Optiksystemfixierorgan 152 gegenüber.

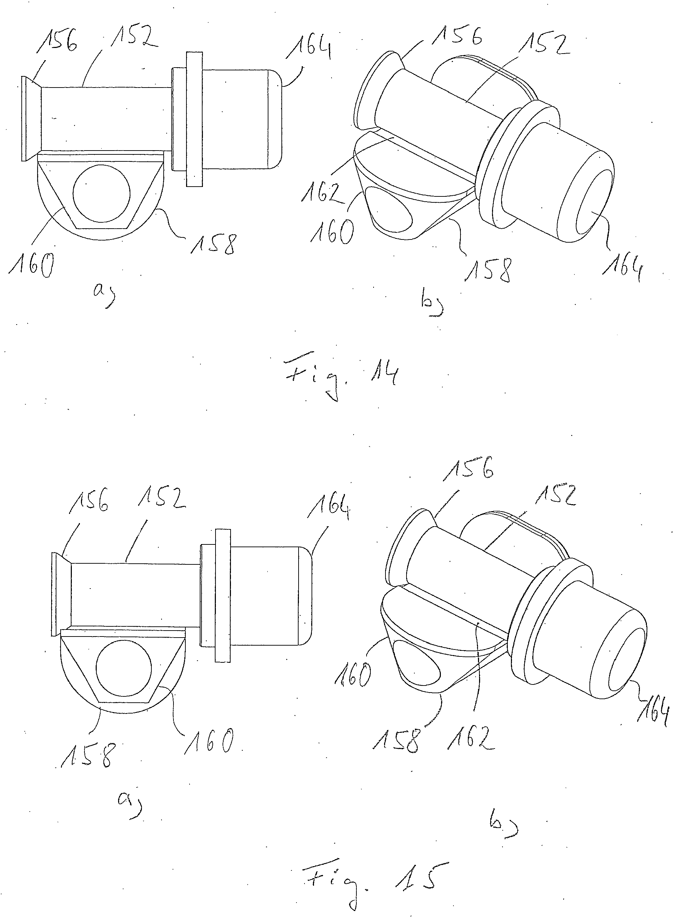

Die Fig. 14 zeigt in ihren Darstellungen a) und b) einen Zustand, bei welchem das Optiksystemfixierorgan 152 entgegen seiner Federvorspannung beispielsweise durch Druck auf einen aus dem Optikträger 36 hervorstehenden Betätigungsknopf 164 verschoben ist, so dass der kegelstumpfartige Fixierbereich 156 aus der Kontur des Schwalbenschwanzinnenprofils 150 heraus bewegt ist. In diesem Zustand kann das Gegen-Fixierorgan 158 in das Schwalbenschwanzinnenprofil 150 eingeschoben werden, bis die in den Fig. 14a) und 14b) erkennbare Relativpositionierung zwischen dem Gegen-Fixierorgan 158 und dem Optiksystemfixierorgan 152 erreicht ist. Wird nachfolgend die Beaufschlagungswirkung aufgegeben, so kehrt das Optiksystemfixierorgan 152 in die in den Fig. 12 und 15a) und 15b) gezeigte Positionierung zurück, in welcher der kegelstumpfartige Fixierbereich 156 in die im Gegen-Fixierorgan 158 gebildete Einsenkung 162 eingreift. Es ist damit zwischen dem Optikträger 36 und dem Gegen-Fixierorgan 158 am Optiksystem 38 ein Formschlusseingriff hergestellt, welcher ein Herausbewegen des Gegen-Fixierorgans 158 aus dem Schwalbenschwanzinnenprofil 150 verhindert. Zum Entfernen des Optiksystems ist es erforderlich, das Optiksystemfixierorgan 152 durch Beaufschlagung in die in den Fig. 14a) und 14b) gezeigte Positionierung zu bringen, also den Eingriff des Fixierbereichs 156 in die Einsenkung 162 aufzuheben. Darauf folgend kann das Gegen-Fixierorgan 158 aus dem Schwalbenschwanzinnenprofil 150 herausgezogen und somit das Optiksystem 38 vom Optikträger 36 losgelöst werden.

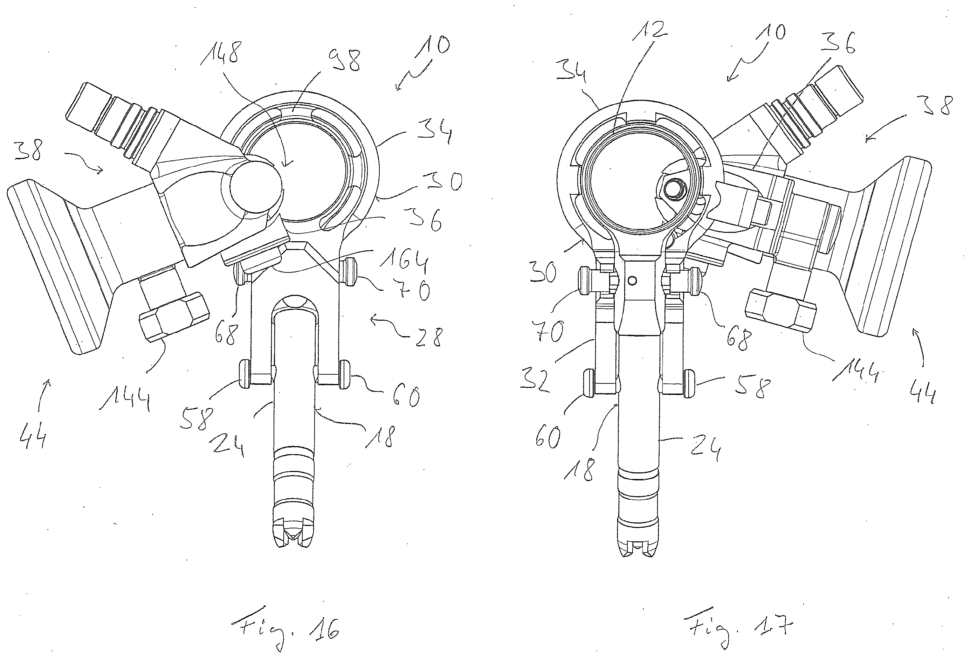

Bei an dem Optikträger 36 fixiertem Optiksystem 38 kann in der vorangehend beschriebenen Art und Weise der Optikträger 36 entlang der Führungsnut 98 am Führungsbahnbereich 34 bewegt werden und beispielsweise in die in den Fig. 16 und 17 dargestellte Positionierung, gleichermaßen jedoch in jedwede beliebige Positionierung entlang der Führungsnut 98 bzw. der dadurch definierten kreisringartigen Bewegungsbahn B gebracht und in jeder beliebigen Positionierung fixiert werden. Im Verlaufe dieser Bewegung bewegt sich der Optikbereich 40 innerhalb des Vorrichtungskörpers 12 gleichermaßen auf einer kreisartigen Bewegungsbahn, jedoch immer nahe an der Innenoberfläche des Vorrichtungskörpers 12, so dass unabhängig von der jeweiligen Positionierung des Optiksystems 38 entlang der Bewegungsbahn B ein vergleichsweise großer und durch das Optiksystem 38 im Wesentlichen nicht beeinträchtigter Querschnitt für das Einführen endoskopischer Instrumente oder dergleichen in einen zu behandelnden Körper erhalten bleibt.

Im Folgenden wird die Vorgehensweise beschrieben, mit welcher die vorangehend beschriebene Vorrichtung 10 bei der Durchführung eines operativen Eingriffs beispielsweise im Wirbelsäulenbereich eines menschlichen oder tierischen Körpers eingesetzt werden kann.

Zunächst wird dort, wo der operative Eingriff vorzunehmen ist, ein Einschnitt der erforderlichen Größe erzeugt. Durch ein Dilatationssystem, welches beispielsweise mehrere Dilatationshülsen mit jeweils bezüglich einander unterschiedlichem Außen- sowie Innendurchmesser umfassen kann, wird dieser Einschnitt zu einer für die Durchführung des operativen Eingriffs erforderlichen Größe aufgeweitet. Dabei werden, beginnend mit einer Dilatationshülse kleineren Durchmessers, sukzessive die Dilatationshülsen übereinander geschoben, bis die gewünschte Öffnungsgröße erreicht ist. Über die letzte in den Einschnitt eingeführte und diesen aufweitende Dilatationshülse des Dilatationssystems wird der Vorrichtungskörper 12 in den zu behandelnden Körper eingeführt, und zwar bis zur gewünschten Tiefe. Dafür können verschieden dimensionierte Vorrichtungskörper 12 bereitgehalten werden, so dass, je nach erforderlicher Öffnungsgröße, nur die minimal erforderliche und somit den behandelten Körper auch minimal belastende Öffnungsgröße erzeugt wird.

Nach dem Einführen des Vorrichtungskörpers 12 in den zu behandelnden Körper kann der Trägerbereich 24 an einem Gelenkstativ arretiert werden, so dass gewährleistet ist, dass während der Durchführung des operativen Eingriffs eine ungewünschte Bewegung des Vorrichtungskörpers 12 und damit der gesamten Vorrichtung 10 nicht auftreten kann.

Während des Einführens des Vorrichtungskörpers 12 in den zu behandelnden Körper ist vorteilhafterweise die Optikträgeranordnung 28 nicht am Schwenkträgerelement 18 vorgesehen, was die Handhabung des Vorrichtungskörpers 12 mit dem daran fest vorgesehenen Schwenkträgerelement 18 erleichtert. Da die Optikträgeranordnung 28 selbst bereits nicht am Vorrichtungskörper 12 vorgesehen ist, ist selbstverständlich auch kein Optiksystem 38 mit dem Vorrichtungskörper 12 verkoppelt.

Ist der Vorrichtungskörper 12 in der erforderlichen Positionierung und vorteilhafterweise in dieser Positionierung fest arretiert, wird die Optikträgeranordnung 28, umfassend das Führungsbahnelement 30 und den Optikträger 36, bei davon noch entkoppeltem Optiksystem 38 zunächst in der Einführrelativpositionierung bezüglich des Schwenkträgerelements 18 positioniert. Das Führungsbahnelement 30 wird mit seinem Schwenk/Arretierbereich mit dem Trägerbereich 24 des Schwenkträgerelements 18 zur Schwenkbewegung verkoppelt und in die Betriebsstellung verschwenkt. Dabei kann vorteilhafterweise unter Einsatz der Arretiervorsprünge 68, 70 die vorangehend besprochene Arretierwirkung erzeugt werden, so dass der Führungsbahnbereich 34 fest und in definierter Positionierung angrenzend an das proximale Ende 16 des Vorrichtungskörpers 12, diesen im Bereich des proximalen Endes 16 ggf. geringfügig übergreifend, positioniert wird.

Nachfolgend kann das Optiksystem 38 in Richtung der Vorrichtungskörperlängsachse L mit seinem Gegen-Fixierorgan 158 bzw. dessen Schwalbenschwanzaußenprofil 160 in das Schwalbenschwanzinnenprofil 150 am Optikträgerkörper 106 eingeführt werden. Im Verlaufe dieser Einführbewegung wird entweder durch manuelle Beaufschlagung oder durch entsprechende Abweisschrägen das Optiksystemfixierorgan 152 in seiner Freigabestellung verschoben, bis die Einsenkung 162 mit dem Optiksystemfixierorgan 152 ausgerichtet ist. In diesem Zustand gelangt das Optiksystemfixierorgan 152 durch Vorspannbeaufschlagung desselben in den Fixierzustand, so dass das Gegen-Fixierorgan 158 am Optikträger 36 fixiert ist. In diesem Zustand liegt der Optikbereich 40 des Optiksystems 38 zumindest bereichsweise innerhalb des Vorrichtungskörpers 12.

Nachfolgend kann der Optikträger 36 mit dem daran getragenen Optiksystem 38 entlang der Bewegungsbahn B, d. h. entlang der Führungsnut 98 im Führungsbahnbereich 34, verschoben werden, so dass das Optiksystem 38 mit seinem Sichtfeld F so positioniert ist, dass optimale Sicht auf den zu behandelnden Bereich besteht. Nun kann das Fixierorgan 122 in seine Fixierstellung gebracht werden, in welcher es von radial außen gegen den Führungsbahnbereich 34 gepresst ist, so dass dieser zwischen dem Fixierorgan 122 und dem in die Führungsnut 98 eingreifenden Führungsvorsprung 104 geklemmt ist. In diesem Zustand ist der Optikträger 36 und mit diesem das Optiksystem 38 gegen Bewegung entlang der Führungsnut 98 fixiert.

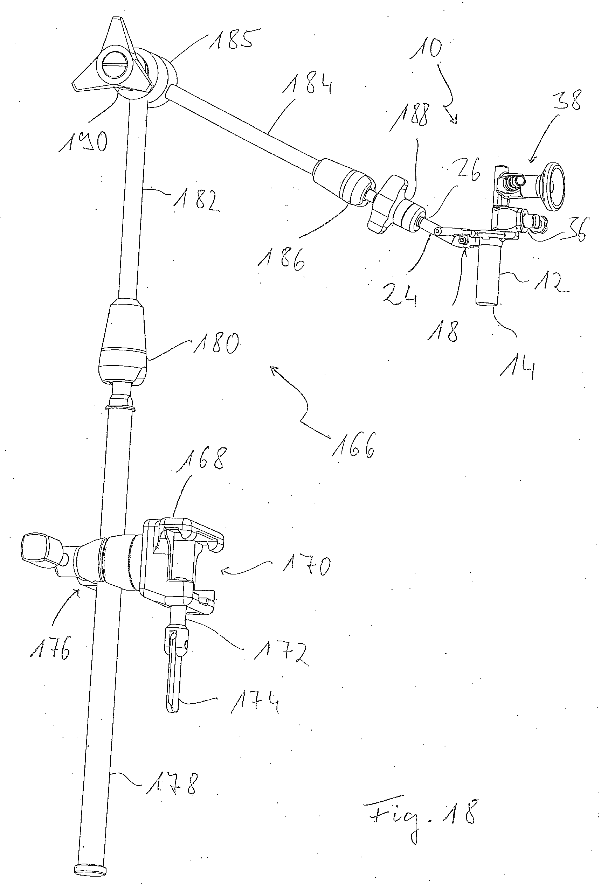

Die Fig. 18 zeigt die Vorrichtung 10 mit dem daran getragenen Optiksystem 38 an einer allgemein auch als Gelenkstativ bezeichneten Trägeranordnung 166. Diese umfasst ein Klemmorgan 168, das mit einem ersten Klemmbereich 170 beispielsweise an einem Operationstisch festgeklemmt werden kann. Hierzu kann eine Klemmschraube 172 vermittels eines daran schwenkbar getragenen Betätigungshebels 174 betätigt werden.

In einem zweiten Klemmbereich 176 kann eine Klemmstange 188 der Trägeranordnung 166 am Klemmorgan 168 geklemmt werden, wobei die Klemmstange 178 bezüglich des Klemmorgans 168 auch zur Einstellung der Höhenpositionierung der Vorrichtung 10 in eine geeignete Verschiebepositionierung gebracht werden kann und dann im zweiten Klemmbereich 176 geklemmt werden kann.

Über ein Kugelgelenk 180 ist eine erste Trägerstange 182 mit der Klemmstange 178 gelenkig verbunden. Eine zweite Trägerstange 184 ist vermittels eines Schwenkgelenks 185 mit der ersten Trägerstange 182 schwenkbar gekoppelt. Über ein weiteres Kugelgelenk 186 ist die zweite Trägerstange 184 mit einem Halteorgan 188 gelenkig verbunden. An dem Halteorgan 188 kann der Trägerbereich 24 des Schwenkträgerelements 18 im Bereich seines freien Endes 26 gehaltert werden.

Durch ein am Schwenkgelenk 186 vorgesehenes und beispielsweise durch Hand betätigbares Klemmorgan 190 können nach Einstellung der gewünschten Positionierung der Vorrichtung 10 die beiden Kugelgelenke 180, 186 sowie auch das Schwenkgelenk 185 gegen Bewegung blockiert werden, so dass in einer dann eingefrorenen Positionierung einerseits die Klemmstange 178 bezüglich der ersten Trägerstange 182, andererseits die zweite Trägerstange 184 bezüglich des Halteorgans 188 und des Weiteren die erste Trägerstange 182 bezüglich der zweiten Trägerstange 184 im wesentlichen unbeweglich gehalten sind. In diesem Zustand ist die Vorrichtung 10 dann mit bereits in dem in dem zu behandelnden Körper gebildeten Einschnitt positioniertem Vorrichtungskörper 12 stabil positioniert und ermöglicht somit die Durchführung des vorzunehmenden operativen Eingriffs.

Während der Durchführung eines operativen Eingriffs kann der Optikträger 36 mit dem daran getragenen Optiksystem 38 erforderlichenfalls durch Bewegung entlang der Führungsnut 98 in eine andere Positionierung gebracht werden. Hierzu wird das Fixierorgan 122 radial in Richtung vom Führungsbahnbereich 34 weg bewegt und die Klemmwirkung des Führungsbahnbereichs 34 zwischen dem Fixierorgan 122 und dem Führungsvorsprung 104 aufgehoben. Nach Bewegung entlang der Bewegungsbahn B bzw. in der Führungsnut 98 und Erreichen der gewünschten neuen Positionierung kann das Fixierorgan 122 dann erneut gegen den Außenumfang des Führungsbahnbereichs 34 gepresst werden und somit der Optikträger 36 fixiert werden.

Nach Beendigung des operativen Eingriffs durch den Vorrichtungskörper 12 hindurch wird zunächst das Optiksystem 38 vom Optikträger 36 entfernt. Hierzu wird das Optiksystemfixierorgan 152 durch Beaufschlagung in seine Freigabestellung gebracht und das Gegen-Fixierorgan 158 aus dem Optikträgerkörper 106 herausgezogen. Ist das Optiksystem 38 entfernt, kann durch Pressen der beiden Arretiervorsprünge 68, 70 gegeneinander deren Arretiereingriff in die Arretieraussparungen 76, 78 aufgehoben werden. Daraufhin kann das Führungsbahnelement 30 aus seiner Betriebsstellung heraus in die Einführrelativpositionierung verschwenkt werden, so dass die Gabelendbereiche 46, 48 von den Schwenkvorsprüngen 58, 60 abgezogen werden können und die Optikträgeranordnung 28 vom Vorrichtungskörper 12 entfernt werden kann. Zuletzt wird der Vorrichtungskörper 12 aus dem im behandelten Körper gebildeten Einschnitt herausgezogen, gefolgt durch die dann noch erforderliche Behandlung im Bereich des Einschnitts, beispielsweise umfassend das Vernähen im Bereich des Einschnitts. Die verschiedenen Bestandteile der Vorrichtung 10, also der Vorrichtungskörper 12 mit dem daran fest getragenen Schwenkträgerelement 18 einerseits und die Optikträgeranordnung 28 mit dem Führungsbahnelement 30 und dem Optikträger 36 andererseits können dann in dem voneinander losgelösten Zustand zur erneuten Wiederverwendung zunächst einer Sterilisation unterzogen werden. Daraufhin kann ein weiterer operativer Eingriff in der vorangehend beschriebenen Art und Weise unter Einsatz bei einer Vorrichtung 10 und erforderlichenfalls eines Dilatationssystems durchgeführt werden.

Wie bereits vorangehend dargelegt, können Vorrichtungskörper 12 mit verschiedenen Abmessungen, also verschiedenen Durchmessern, und ggf. auf diese verschiedenen Größen abgestimmte Optikträgeranordnungen 28 in einem Vorrichtungssatz bereitgehalten werden, um, je nach erforderlicher Größe der Zugriffsöffnung jeweils den Vorrichtungskörper 12 und die zugeordnete Optikträgeranordnung 28 zum Einsatz bringen zu können.

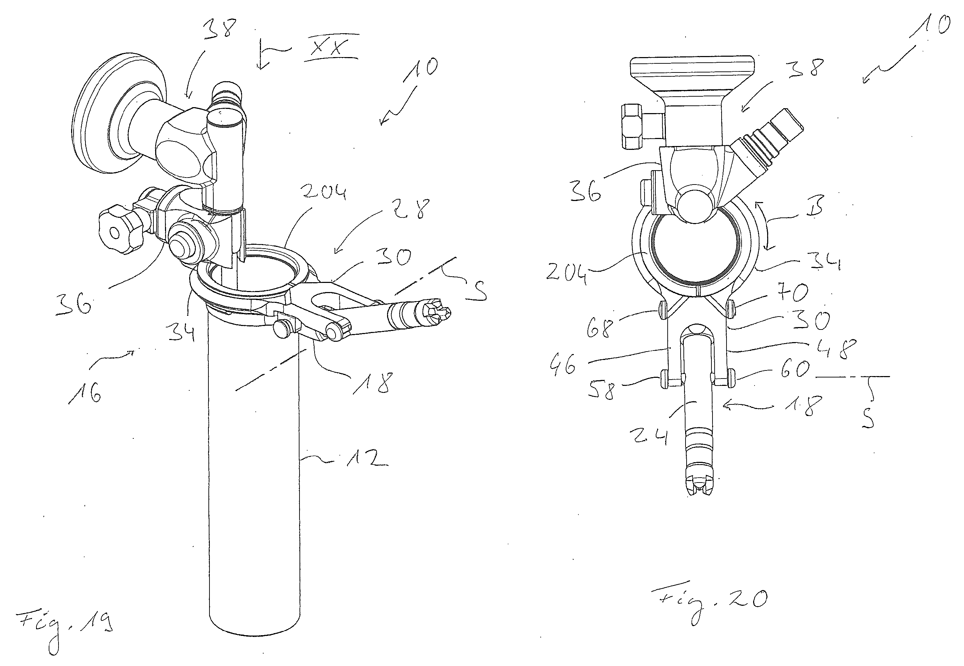

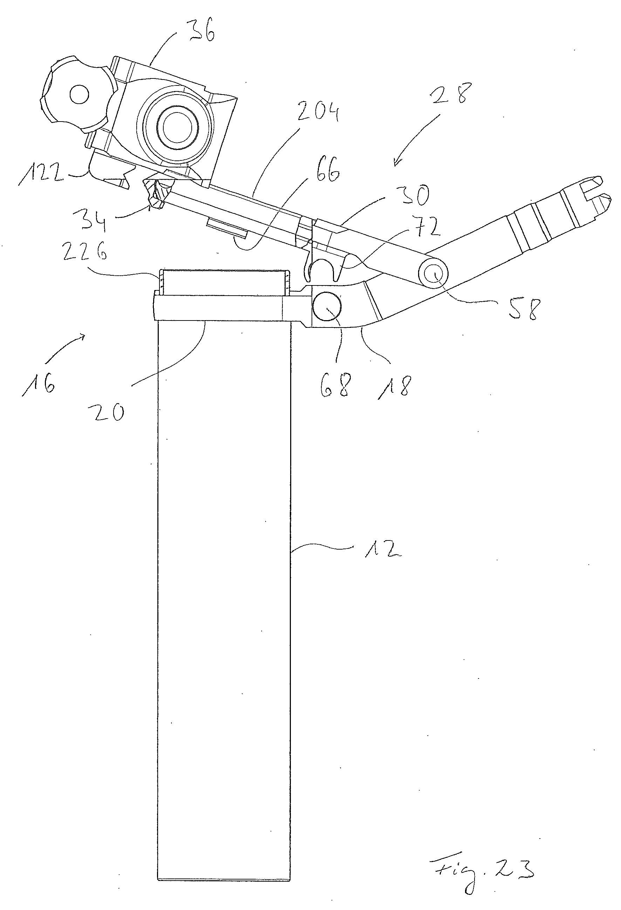

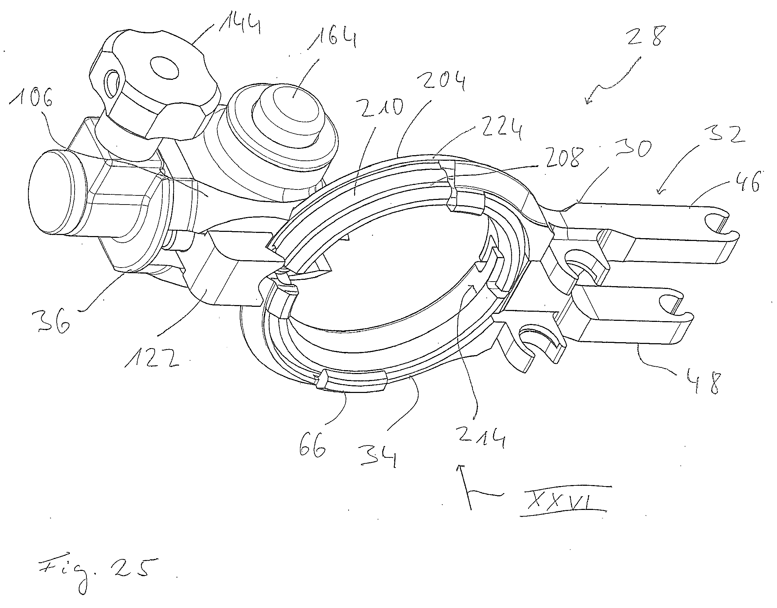

Mit Bezug auf die Fig. 19 bis 29 wird nachfolgend eine alternative Ausgestaltungsform der Vorrichtung 10 zum Bereitstellen einer Zugriffsöffnung in einen Körper beschrieben. Diese Ausgestaltungsform entspricht weitestgehend der vorangehend hinsichtlich ihres konstruktiven Aufbaus und hinsichtlich ihres Einsatzes bei der Durchführung eines operativen Eingriffs beschriebenen Vorrichtung 10. Dies betrifft insbesondere auch die grundsätzliche Ausgestaltung der Optikträgeranordnung 28 mit ihrem gabelartig ausgebildeten Führungsbahnelement 30 und dessen Schwenkwechselwirkung mit dem Schwenkträgerelement 18. Auch der Optikträger 36 ist insbesondere hinsichtlich der zur Fixierung desselben am Führungsbahnbereich 34 und daran zur Fixierung eines Optiksystems 38 daran vorgesehenen technischen Maßnahmen im Wesentlichen so ausgestaltet, wie vorangehend dargelegt, ebenso wie der rohrartig ausgebildete Vorrichtungskörper 12 und das an diesen festgelegte Schwenkträgerelement 18. Es wird daher hinsichtlich des grundsätzlichen konstruktiven Aufbaus und der Funktionalität dieser Baugruppen auf die vorangehenden Ausführungen verwiesen. In der folgenden Beschreibung der bei der Ausgestaltungsform der Fig. 19 bis 29 bezüglich der vorangehend beschriebenen Ausgestaltungsform wesentlichen Unterschiede in der konstruktiven Ausführung sind Bauteile bzw. Baugruppen, welche vorangehend beschriebenen Bauteilen bzw. Baugruppen entsprechen, mit dem gleichen Bezugszeichen bezeichnet.

Auch bei der in den Fig. 19 bis 29 dargestellten Ausgestaltungsform der Vorrichtung 10 ist der Führungsbahnbereich 34 des Führungsbahnelements 30 grundsätzlich ringartig ausgebildet, so dass bei Positionierung des Führungsbahnelements 30 in seiner in Fig. 19 auch erkennbaren Betriebsstellung durch Verschwenkung um die Schwenkachse S der Führungsbahnbereich 34 im Bereich des proximalen Endes 16 des Vorrichtungskörpers 12 positioniert ist bzw., wie im Folgenden auch dargelegt, in diesem Bereich den Vorrichtungskörper 12 radial außen bezüglich der Vorrichtungskörperlängsachse L umgibt.

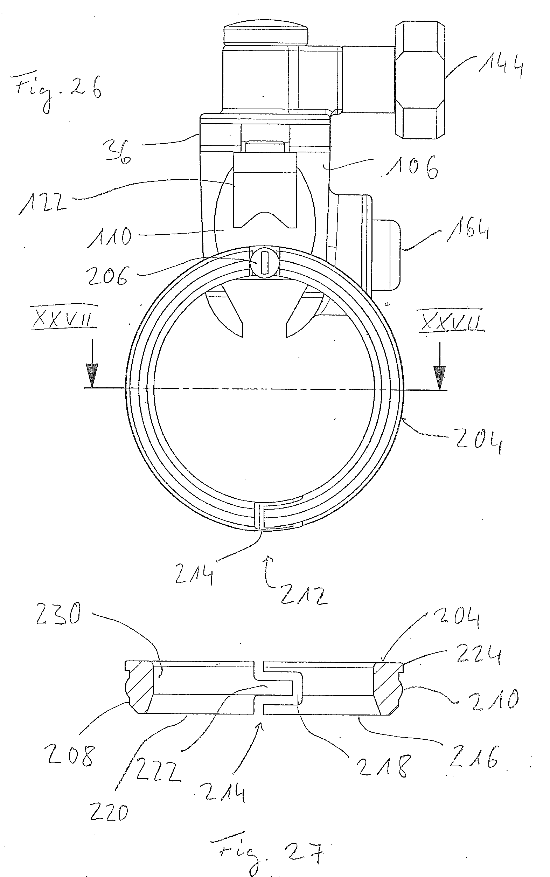

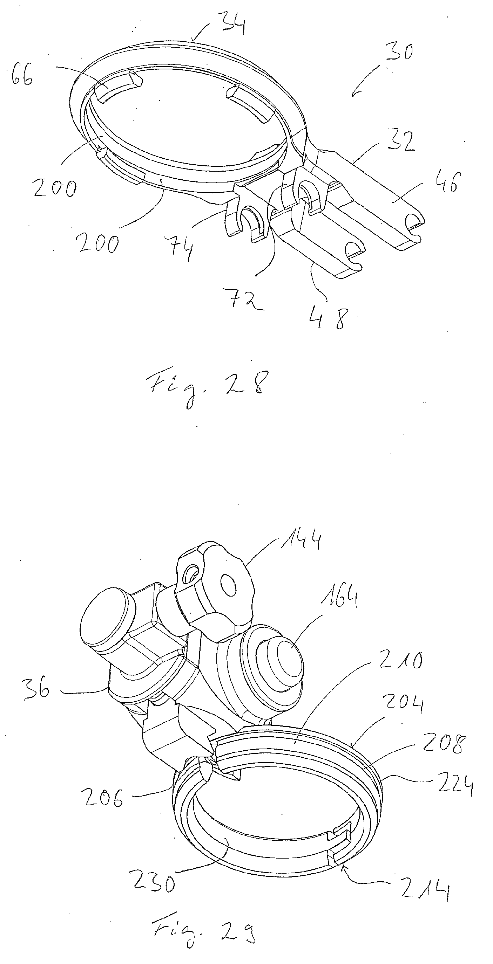

Das Führungsbahnelement 30 weist in seinem ringartig ausgebildeten Führungsbahnbereich 34 eine in der Betriebsstellung der Optikträgeranordnung 28 die Vorrichtungskörperlängsachse L vorzugsweise vollständig ringartig umgebende und als Innenumfangsfläche ausgebildete Führungsfläche 200 als erste Führungsformation 97 auf. In dieser Führungsfläche 200 ist eine vorteilhafterweise sich ebenfalls ringartig geschlossen in Richtung der kreisartigen Bewegungsbahn B sich erstreckende Führungseinsenkung 202 vorgesehen. Die zweite Führungsformation 103 umfasst einen Führungsring 204, der, wie dies in Fig. 26 und in Fig. 29 deutlich zu erkennen ist, durch ein Befestigungsorgan 206, beispielsweise Schraubbolzen oder dergleichen, am Optikträgerkörper 106 an dessen dem Führungsbahnbereich 34 zugewandt positionierter Seite 110 festgelegt ist. Der Führungsring 204 weist eine als Außenumfangsfläche ausgebildete, vorzugsweise im Wesentlichen geschlossen ringartig ausgebildete Gegen-Führungsfläche 208 auf. An dieser Gegen-Führungsfläche 208 ist nach radial außen vorspringend ein in Richtung der kreisartigen Bewegungsbahn B sich erstreckender, wulstartiger oder höckerartiger Führungsvorsprung 210 ausgebildet, dessen Querschnittsgeometrie zur Querschnittsgeometrie der Führungseinsenkung 202 im Wesentlichen komplementär ist.

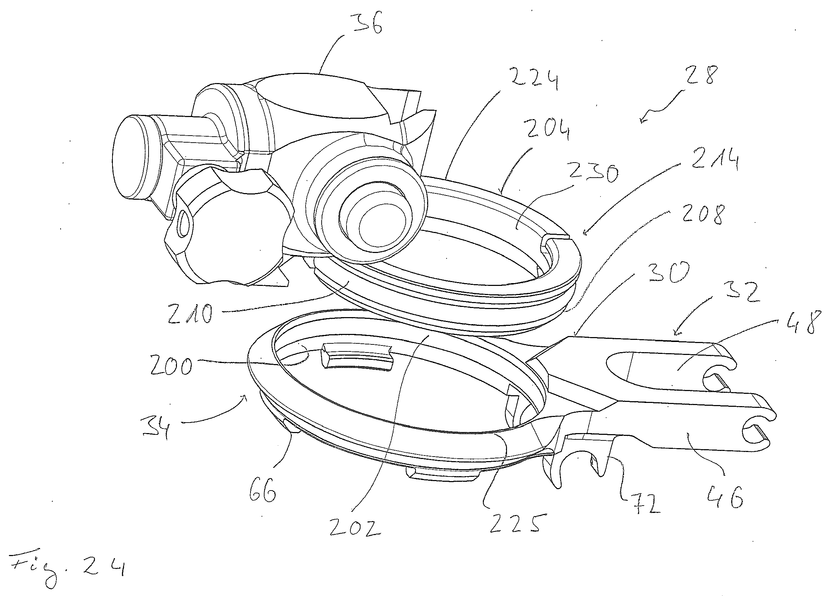

Man erkennt in den Fig. 24, 26 und 27 weiter, dass der Führungsring 204 grundsätzlich zwar ringartig geschlossen ist, in einem Umfangsbereich 212 jedoch eine Unterbrechung 214 aufweist. In diesem Umfangsbereich 212 ist beispielsweise in einem Umfangsabschnitt 216 des Führungsrings 204 eine in Umfangsrichtung offene Einsenkung 218 ausgebildet. In einem gegenüberliegenden Umfangsabschnitt 220 ist ein in die Einsenkung 218 eingreifender, in Umfangsrichtung vorstehender Vorsprung 222 ausgebildet, so dass der Führungsring 204 insbesondere in axialer Ansicht im Wesentlichen vollständig umlaufend ausgebildet ist. Zwischen den beiden Umfangsabschnitten 216, 220 ist ein Umfangszwischenraum gebildet, welcher es ermöglicht, durch Belastung die beiden Umfangsabschnitte 216, 220 einander anzunähern. Der Führungsring 204 ist somit grundsätzlich radialelastisch ausgebildet, so dass bei Radialbelastung dessen Außenabmessung verringert werden kann. Insbesondere kann diese Verringerung der Außenabmessung in derartigem Ausmaß erfolgen, dass in diesem komprimierten Zustand der Führungsring 204 mit seinem Führungsvorsprung 210 in den Führungsbahnbereich 34 eingeführt werden kann, bis der Führungsvorsprung 210 mit der Führungseinsenkung 202 ausgerichtet ist, dieser also gegenüberliegt. Um diese Relativpositionierung definiert vorgeben zu können, sind beispielsweise die an mehreren Umfangspositionen des Führungsbahnelements 30 vorgesehenen Positioniervorsprünge 66 so ausgebildet, dass sie bezüglich der Führungsfläche 200 nach radial innen vorstehen. Des Weiteren kann am Führungsring 204 ein bezüglich der Gegen-Führungsfläche 208 nach radial außen vorspringender Bundbereich 224 vorgesehen sein, der an einer Stirnseite 225 des Führungsbahnbereichs 34 zur Anlage kommen kann.

Ist der Führungsring 204 in maximalem Ausmaß in die im Führungsbahnbereich 34 gebildete Öffnung eingeführt, kann die Kompressionsbelastung aufgegeben werden, so dass der Führungsring 204 aufgrund seiner inhärenten Radialelastizität wieder in seine Ausgangsform zurückkehrt, d. h., sich radial aufweitet. Dabei tritt der Führungsvorsprung 210 in die Führungseinsenkung 202 ein und die Gegen-Führungsfläche 208 kommt zumindest bereichsweise in Anlage an der Führungsfläche 200.

Um diese Radialelastizität des Führungsrings 204 erreichen zu können, ist der Führungsring 204 vorteilhafterweise mit einem Material aufgebaut, das einerseits beispielsweise bei manueller Einwirkung eine ausreichend starke Verformung zulässt, das andererseits aber wieder in seine Ausgangsform zurückkehren kann, also bei der erforderlichen Kompression nicht in einen plastisch verformten Zustand übergeht. Des Weiteren muss dieses Material für die Durchführung operativer Eingriffe und die danach auch erforderliche Sterilisierung beispielsweise bei vergleichsweise hohen Temperaturen geeignet sein. Als Aufbaumaterial für den Führungsring 204 kann beispielsweise PEEK-Material eingesetzt werden.

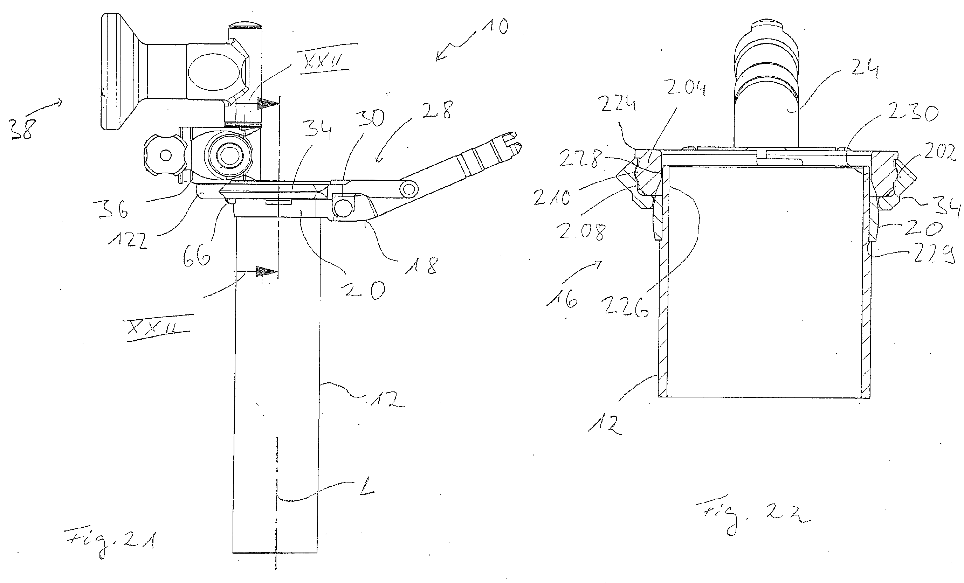

Um bei dem Aufbau der Fig. 19 bis 29 eine noch weiter verbesserte Führungsfunktion für den Optikträger 36 bzw. den daran vorgesehenen Führungsring 204 erreichen zu können, ist im Bereich des proximalen Endes 16 des Vorrichtungskörpers 12 ein beispielsweise zylindrischer oder rohrartiger Führungsabschnitt 226 mit einer als Außenumfangsfläche ausgebildeten weiteren Führungsfläche 228 vorgesehen. Dieser Führungsabschnitt 226 kann, wie dies die Fig. 22 zeigt, durch einen Endabschnitt des Vorrichtungskörpers 12 im Bereich des proximalen Endes 16 bereitgestellt sein, also mit dem Vorrichtungskörper 12 integral ausgebildet sein. Beispielsweise kann der Vorrichtungskörper 12 im Bereich seines proximalen Endes 16 mit beispielsweise stufenartig verringerter Wandungsstärke ausgebildet sein, so dass einerseits der Führungsabschnitt 226 gebildet ist, andererseits ein Positionierungsanschlag 229 für den ringartig ausgebildeten Befestigungsbereich 20 des Schwenkträgerelements 18 gebildet ist.

Am Führungsring 204 ist als Innenumfangsfläche ausgebildet eine weitere Gegen-Führungsfläche 230 vorgesehen, die bei in der Betriebsstellung positionierter Optikträgeranordnung 28 den Führungsabschnitt 226 am proximalen Ende 16 des Vorrichtungskörpers 12 radial außen umgibt bzw. in Führungsanlage daran kommen kann. Somit ist in der Betriebsstellung der Führungsring 204 radial zwischen dem Führungsbahnbereich 34 bzw. dessen Führungsfläche 200 und dem Führungsabschnitt 226 bzw. dessen weiterer Führungsfläche 228 positioniert. Wie vorangehend bereits dargelegt, umgibt in dieser Positionierung der Führungsbahnbereich 34 den Führungsabschnitt 226 und damit bei der dargestellten integralen Ausgestaltung desselben mit dem Vorrichtungskörper 12 diesen Vorrichtungskörper 12 im Bereich seines proximalen Endes 16. Auch bei dieser Ausgestaltung ist gemäß den Prinzipien der vorliegenden Erfindung in der Betriebsstellung der Führungsbahnbereich 34 im Bereich des proximalen Endes 16, also anschließend an das proximale Ende 16 des Vorrichtungskörpers 12 positioniert, obgleich hier ein axialer Überlapp zwischen diesen beiden Baugruppen besteht. Dieser axiale Überlapp ist vergleichsweise kurz. Wie die Fig. 22 dies zeigt, überlappt der Führungsbahnbereich 34 sich beispielsweise nicht vollständig mit dem Vorrichtungskörper 12, ebenso wie der Führungsring 204.

Zur Fixierung des Optikträgers 36 am Führungsbahnbereich 34 des Führungsbahnelements 30 kann, wie vorangehend detailliert erläutert, das am Optikträger 36 vorgesehene Fixierorgan 122 von radial außen gegen den auch bei dieser Ausgestaltung beispielsweise mit V-artiger, konvexer Profilierung ausgebildeten Führungsbahnbereich 34 gepresst werden. Dabei werden in dem Umfangsbereich, in welchem das Fixierorgan 122 von radial außen gegen den Führungsbahnbereich 34 gepresst wird, die Führungsfläche 200 und die Gegen-Führungsfläche 208 verstärkt gegeneinander gepresst, so dass letztendlich der Führungsbahnbereich 34 radial zwischen dem am Optikträger 36 vorgesehenen Fixierorgan 122 und dem ebenfalls am Optikträger 36 vorgesehenen Führungsring 204 geklemmt wird. Zur Verstellung des Optikträgers 36 mit dem daran getragenen Optiksystem 38 entlang der Bewegungsbahn B kann diese Klemmwirkung durch radiales Wegbewegen des Fixierorgans 122 vom Führungsbahnbereich 34 aufgehoben werden, woraufhin der Führungsring 204 im Führungsbahnbereich 34 zur Drehung um die Vorrichtungskörperlängsachse L freigegeben ist und somit der Optikträger 36 mit dem daran getragenen Optiksystem 38 in beliebiger anderer Umfangspositionierung positioniert werden kann.

Der vorangehend beschriebene und in den Fig. 19 bis 29 dargestellte Aufbau der Vorrichtung 10 insbesondere im Bereich der Optikträgeranordnung 28 bringt verschiedene substantielle Vorteile mit sich. Zum einen besteht zwischen dem Führungsbahnelement 30 bzw. dem Führungsbahnbereich 34 desselben und dem am Optikträger 36 vorgesehenen Führungsring 204 eine im Wesentlichen ringartig geschlossene Führungswechselwirkung. Dies bedeutet, dass auch dann, wenn am Optikträger 36 ein Optiksystem 38 getragen ist und aufgrund dessen Gewichtsbelastung ein Kippmoment am Optikträger 36 entsteht, keine Verkantung zwischen der ersten Führungsformation 97, also der am Führungsbahnbereich 34 vorgesehenen Führungsfläche 200, und der zweiten Führungsformation 103, also dem am Optikträger 36 vorgesehenen Führungsring 204 mit seiner Gegen-Führungsfläche 208 entstehen kann. Dieser Effekt wird besonders effizient dann erreicht, wenn, wie dies im vorliegenden Falle dargestellt ist, der Führungsring 204 im Wesentlichen vollständig ringartig geschlossen ist. Grundsätzlich sei jedoch darauf hingewiesen, dass dieser Effekt auch erreicht werden kann, wenn der Führungsring 204 als Ringsegment ausgebildet ist, beispielsweise mit einem Umfangserstreckungswinkel von mehr als 180°.

Ein weiterer substantieller Vorteil liegt darin, dass mit den beiden in Führungswechselwirkung tretenden Flächen, nämlich der Führungsfläche 200 und der Gegen-Führungsfläche 208, Formationen bereitgestellt sind, welche keine tieferen Nuten oder Nuthinterschneidungsbereiche, also keine schwer zugänglichen Volumenbereiche beispielsweise im Inneren des Führungsbahnbereichs 34 aufweisen. Dies ist insbesondere bei der Sterilisierung der Vorrichtung 10 nach Durchführung eines operativen Eingriffs von Vorteil, da die Gefahr, dass schwer zugängliche Innenvolumenbereiche nicht ausreichend sterilisiert werden, nicht besteht.

## Patentansprüche

1. Vorrichtung zum Bereitstellen einer Zugriffsöffnung in einen Körper, insbesondere für eine Wirbelsäulenoperation, umfassend einen entlang einer Vorrichtungskörperlängsachse (L) sich erstreckenden, rohrartigen Vorrichtungskörper (12) mit einem innerhalb eines Körpers zu positionierenden distalen Ende (14) und einem außerhalb eines Körpers zu positionierenden proximalen Ende (16) sowie eine Optikträgeranordnung (28), wobei die Optikträgeranordnung (28) im Bereich des proximalen Endes (16) mit einem auf einer Bewegungsbahn (B) um die Vorrichtungskörperlängsachse (L) bewegbaren Optikträger (36) positioniert oder positionierbar ist,
**dadurch gekennzeichnet, dass** die Optikträgeranordnung (28) ein den Optikträger (36) tragendes Führungsbahnelement (30) mit einem durch Verschwenkung um eine Schwenkachse (S) in einer Betriebsstellung im Bereich des proximalen Endes (16) des Vorrichtungskörpers (12) positionierbaren Führungsbahnbereich (34) umfasst.

2. Vorrichtung nach Anspruch 1,
**dadurch gekennzeichnet, dass** der Führungsbahnbereich (34) ringartig ausgebildet ist und eine ringartige erste Führungsformation (97), vorzugsweise Führungsnut (98) oder Führungsfläche (200), aufweist, wobei eine zweite Führungsformation (103), vorzugsweise Führungsvorsprung (104) oder Führungsring (204), des Optikträgers (36) zum Bewegen des Optikträgers (36) auf seiner Bewegungsbahn (B) um die Vorrichtungskörperlängsachse (L) entlang der ringartigen ersten Führungsformation (98) geführt ist und an vorzugsweise beliebiger Positionierung entlang der ersten Führungsformation (98) fixierbar ist.

3. Vorrichtung nach Anspruch 1 oder 2,
**dadurch gekennzeichnet, dass** das Führungsbahnelement (30) einen Schwenk/Arretierbereich (32) aufweist zur schwenkbaren Anbringung und Arretierung des Führungsbahnelements (30) in der Betriebsstellung an einem von dem Vorrichtungskörper (12) sich weg erstreckenden Schwenkträgerelement (18).

4. Vorrichtung nach Anspruch 3,
**dadurch gekennzeichnet, dass** das Schwenkträgerelement (18) einen den Vorrichtungskörper (12) vorzugsweise ringartig umgebenden und vorzugsweise mit dem proximalen Ende (16) des Vorrichtungskörpers (12) bündig abschließenden Befestigungbereich (20) und einen von dem Befestigungsbereich (20) sich weg erstreckenden Trägerbereich (24) umfasst.

5. Vorrichtung nach Anspruch 3 oder 4,
**dadurch gekennzeichnet, dass** an einer Baugruppe von Schwenkträgerelement (18) und Schwenk/Arretierbereich (32), vorzugsweise dem Schwenkträgerelement (18), wenigstens ein Schwenkvorsprung (58, 60) vorgesehen ist und dass an der anderen Baugruppe von Schwenkträgerelement (18) und Schwenk/Arretierbereich (32), vorzugsweise dem Schwenk/Arretierbereich (32) des Führungsbahnelements (30), in Zuordnung zu jedem Schwenkvorsprung (58, 60) eine zum Einführen eines Schwenkvorsprungs (58, 60) in einem Umfangsbereich offene Schwenkaussparung (50, 52) vorgesehen ist.

6. Vorrichtung nach Anspruch 5,
**dadurch gekennzeichnet, dass** der wenigstens eine Schwenkvorsprung (58, 60) zum Einführen in eine Schwenkaussparung (50, 52) in einer Einführrelativpositionierung zwischen dem Schwenk/Arretierbereich (32) und dem Schwenkträgerelement (18) in wenigstens einem Umfangsbereich eine geringere Querschnittsabmessung aufweist, als in einem anderen Umfangsbereich, wobei bei in einer Schwenkaussparung (50, 52) aufgenommenem Schwenkvorsprung (58, 60) und aus der Einführrelativpositionierung bezüglich einander verschwenktem Schwenk/Arretierbereich (32) und Schwenkträgerelement (18) der wenigstens eine Schwenkvorsprung (58, 60) nicht aus der Schwenkaussparung (50, 52) herausbewegbar ist.

7. Vorrichtung nach Anspruch 5 oder 6,
**dadurch gekennzeichnet, dass** an dem Schwenkträgerelement (18) zwei sich im Wesentlichen entlang einer gemeinsamen Schwenkachse (S) erstreckende Schwenkvorsprünge (58, 60) vorgesehen sind und dass der Schwenk/Arretierbereich (32) des Führungsbahnelements (30) gabelartig mit zwei Gabelendbereichen (46, 48) ausgebildet ist und in jedem Gabelendbereich (46, 48) eine Schwenkaussparung (50, 52) aufweist.

8. Vorrichtung nach einem der Ansprüche 3 bis 7,
**dadurch gekennzeichnet, dass** an einer Baugruppe von Schwenkträgereelement (18) und Schwenk/Arretierbereich (32), vorzugsweise dem Schwenkträgerelement (18), wenigstens ein Arretiervorsprung (68, 70) vorgesehen ist und dass an der anderen Baugruppe von Schwenkträgerelement (18) und Schwenk/Arretierbereich (32), vorzugsweise dem Schwenk/Arretierbereich (32) des Führungsbahnelements (30), in Zuordnung zu jedem Arretiervorsprung (68, 70) eine zum Einführen eines Arretiervorsprungs (68, 70) in einem Umfangsbereich offene Arretieraussparung (76, 78) vorgesehen ist.

9. Vorrichtung nach Anspruch 8,
**dadurch gekennzeichnet, dass** der wenigstens eine Arretiervorsprung (68, 70) in Richtung einer Vorsprungslängsachse (A) zwischen einer Aufnahmestellung zum Einführen in eine Arretieraussparung (76, 78) und einer Arretierstellung bewegbar ist, wobei in der Arretierstellung ein Arretierabschnitt (90, 92) des Arretiervorsprungs (68, 70) in einen Gegen-Arretierabschnitt (94, 96) der Arretieraussparung (76, 78) derart eingreift, dass der Arretiervorsprung (68, 70) nicht aus der Arretieraussparung (76, 78) herausbewegbar ist.

10. Vorrichtung nach Anspruch 9,
**dadurch gekennzeichnet, dass** der wenigstens eine Arretiervorsprung (68, 70) in seine Arretierstellung vorgespannt ist.

11. Vorrichtung nach Anspruch 9 oder 10,
**dadurch gekennzeichnet, dass** am Schwenkträgerelement (18) zwei sich entlang einer gemeinsamen Vorsprungslängsachse (A) erstreckende Arretiervorsprünge (68, 70) vorgesehen sind und dass am Schwenk/Arretierbereich (32) des Führungsbahnelements (30) zwei jeweils eine Arretieraussparung (76, 78) aufweisende Arretieraussparungsvorsprünge (72, 74) vorgesehen sind.

12. Vorrichtung nach einem der Ansprüche 1 bis 11,
**dadurch gekennzeichnet, dass** die Optikträgeranordnung (28) einen am proximalen Ende (16) des Vorrichtungskörpers (12) positionierten oder positionierbaren Führungsbahnbereich (34) umfasst mit einer die Vorrichtungskörperlängsachse (L) ringartig umgebenden ersten Führungsformation (97), vorzugsweise Führungsnut (98) oder Führungsfläche (200), in einer Baugruppe von Führungsbahnbereich (34) und Optikträger (36), vorzugsweise dem Führungsbahnbereich (34), und mit einer zum Bewegen des Optikträgers (36) auf seiner Bewegungsbahn (B) um die Vorrichtungskörperlängsachse (L) entlang der ersten Führungsformation (97) geführten zweiten Führungsformation (103), vorzugsweise Führungsvorsprung (104) oder Führungsring (204), an der anderen Baugruppe von Führungsbahnbereich (34) und Optikträger (36), vorzugsweise dem Optikträger (36).

13. Vorrichtung nach Anspruch 12,
**dadurch gekennzeichnet, dass** die erste Führungsformation (97) eine Führungsnut (98) und die zweite Führungsformation (103) einen Führungsvorsprung (104) umfasst, wobei die Führungsnut (98) zum Eingriff des Führungsvorsprungs (104) in Richtung der Vorrichtungskörperlängsachse (L) vom proximalen Endbereich (16) des Vorrichtungskörpers (12) weg offen ist.

14. Vorrichtung nach Anspruch 13,
**dadurch gekennzeichnet, dass** die Führungsnut (98) einen Nuthinterschneidungsbereich (100) aufweist und dass am Führungsvorsprung (104) ein in die den Nuthinterschneidungsbereich (100) eingreifender Hinterschneidungseingriffsbereich (116) vorgesehen ist.

15. Vorrichtung nach Anspruch 12,
**dadurch gekennzeichnet, dass** die erste Führungsformation (97) eine Führungsfläche (200) und die zweite Führungsformation (103) einen Führungsring (204) umfasst, wobei der Führungsring (204) eine Gegen-Führungsfläche (208) zur Anlage an der Führungsfläche (200) umfasst.

16. Vorrichtung nach Anspruch 15,
**dadurch gekennzeichnet, dass** eine Fläche von Führungsfläche (200) und Gegen-Führungsfläche (208) eine Innenumfangsfläche und die andere Fläche eine Außenumfangsfläche ist.

17. Vorrichtung nach Anspruch 15 oder 16,
**dadurch gekennzeichnet, dass** in Zuordnung zu einer Fläche von Führungsfläche (200) und Gegen-Führungsfläche (208) eine in Richtung der Bewegungsbahn (B) sich erstreckende Führungseinsenkung (202) und in Zuordnung zu der anderen Fläche ein in Richtung der Bewegungsbahn (B) sich erstreckender Führungsvorsprung (210) zum Eingriff in die Führungseinsenkung (206) vorgesehen ist.

18. Vorrichtung nach einem der Ansprüche 15 bis 17,
**dadurch gekennzeichnet, dass** der Führungsring (204) radialelastisch ist.

19. Vorrichtung nach einem der Ansprüche 15 bis 18,
**dadurch gekennzeichnet, dass** im Bereich des proximalen Endes (16) des Vorrichtungskörpers (12) eine die Vorrichtungskörperlängsachse (L) ringartig umgebende weitere Führungsfläche (228) vorgesehen ist und dass am Führungsring (204) eine weitere Gegen-Führungsfläche zur (230) Anlage an der weiteren Führungsfläche (228) vorgesehen ist.

20. Vorrichtung nach einem der Ansprüche 12 bis 19,
**dadurch gekennzeichnet, dass** eine Baugruppe von Führungsbahnbereich (34) und Optikträger (36), vorzugsweise der Optikträger (36), ein gegen die andere Baugruppe von Führungsbahnbereich (34) und Optikträger (36), vorzugsweise den Führungsbahnbereich (34), pressbares Fixierorgan (122) zum Fixieren des Optikträgers (36) vorzugsweise in beliebiger Positionierung entlang der Führungsnut (98) bezüglich des Führungsbahnbereichs (34) umfasst.

21. Vorrichtung nach Anspruch 20,
**dadurch gekennzeichnet, dass** an dem Optikträger (36) ein bezüglich der Vorrichtungskörperlängsachse (L) im Wesentlichen von radial außen gegen einen Widerlagerbereich am Führungsbahnbereich (34) pressbares Fixierorgan (122) vorgesehen ist.

22. Vorrichtung nach Anspruch 21,
**dadurch gekennzeichnet, dass** zum Herstellen und Aufheben der Fixierung das Fixierorgan (122) im Wesentlichen radial bezüglich der Vorrichtungskörperlängsachse (L) bewegbar ist.

23. Vorrichtung nach Anspruch 20, 21 oder 22,
**dadurch gekennzeichnet, dass** dem Fixierorgan (122) ein Fixierorganstellantrieb (130, 138, 142) zum Verstellen des Fixierorgans (122) zwischen einer Fixierstellung und einer Freigabestellung zugeordnet ist.

24. Vorrichtung nach Anspruch 23,
**dadurch gekennzeichnet, dass** der Fixierorganstellantrieb (130, 138, 142) einen mit dem Fixierorgan (122) bewegbaren ersten Beaufschlagungsbereich (134) und einen den ersten Beaufschlagungsbereich (134) beaufschlagenden und zum Bewegen des Fixierorgans (122) bezüglich des ersten Beaufschlagungsbereichs (134) verlagerbaren zweiten Beaufschlagungsbereich (140) an einem Beaufschlagungsorgan (138) umfasst, wobei wenigstens ein Beaufschlagungsbereich (134, 140), vorzugsweise jeder Beaufschlagungsbereich (134, 140), keilartig ausgebildet ist.

25. Vorrichtung nach Anspruch 24,
**dadurch gekennzeichnet, dass** der erste Beaufschlagungsbereich (134) mit dem Fixierorgan (122) in einer ersten Verschieberichtung (R₁) verschiebbar ist und dass der zweite Beaufschlagungsbereich (140) mit dem Beaufschlagungsorgan (138) in einer zu der ersten Verschieberichtung (R₁) angewinkelten, vorzugsweise dazu im Wesentlichen orthogonal stehenden zweiten Verschieberichtung (R₂) verschiebbar ist.

26. Vorrichtung nach Anspruch 24 oder 25,
**dadurch gekennzeichnet, dass** dem Beaufschlagungsorgan (138) ein Gewindeantrieb (142) zugeordnet ist.

27. Vorrichtung nach einem der Ansprüche 24 bis 26,
**dadurch gekennzeichnet, dass** der erste Beaufschlagungsbereich (134) und der zweite Beaufschlagungsbereich (140) in Formschlusseingriff miteinander stehen.

28. Vorrichtung nach einem der Ansprüche 1 bis 27,
**dadurch gekennzeichnet, dass** an dem Optikträger (36) ein Optikfixierbereich (148) zum Fixieren eines Optiksystems (38) an dem Optikträger (36) vorgesehen ist.

29. Vorrichtung nach Anspruch 28,
**dadurch gekennzeichnet, dass** der Optikfixierbereich (148) ein Schwalbenschwanzinnenprofil (150) oder ein Schwalbenschwanzaußenprofil (160), vorzugsweise Schwalbenschwanzinnenprofil (150), sowie ein zum Herstellen und Aufheben der Fixierung des Optiksystems (38) bezüglich des Optikträgers (36) bewegbares Optiksystemfixierorgan (152) am Optikträger (36) umfasst.

## Claims

1. Device for providing an access opening to a body, in particular for spinal surgery, comprising a tubular device body (12) extending along a longitudinal axis of the device body (L) with a distal end (14) to be positioned inside a body and a proximal end (16) to be positioned outside a body as well as an optics carrier arrangement (28), wherein the optics carrier arrangement (28) is positioned or can be positioned in the region of the proximal end (16) with an optics carrier (36) movable on a movement path (B) about the longitudinal axis of the device body (L),
**characterized by** said optics carrier arrangement (28) comprising a guiding path element (30) carrying the optics carrier (36) with a guiding path section (34) which by pivoting about a pivot axis (S) can be positioned in an operation position in the region of the proximal end (16) of the device body (12).

2. Device according to claim 1,
**characterized by** said guiding path section (34) being adapted ring-like and comprising a ring-like first guiding structure (97), preferably a guiding groove (98) or a guiding surface (200), wherein a second guiding structure (103), preferably a guiding projection (104) or a guiding ring (204), of the optics carrier (36) is guided along the ring-like first guiding structure (98) for moving the optics carrier (36) on its movement path (B) about the longitudinal axis of the device body (L) and wherein it can be fixed preferably at any position along the first guiding structure (98).

3. Device according to claim 1 or 2,
**characterized by** said guiding path element (30) comprising a pivoting/locking section (32) for pivotably mounting and locking the guiding path element (30) in the operation position at a pivot carrier element (18) extending away from said device body (12).

4. Device according to claim 3,
**characterized by** said pivot carrier element (18) comprising a mounting region (20) preferably surrounding the device body (12) in a ring-like manner and ending flush with the proximal end (16) of the device body (12) and comprising a support region (24) extending away from the mounting region (20).

5. Device according to claim 3 or 4,
**characterized by** at least one pivot projection (58, 60) being provided at one assembly out of pivot carrier element (18) and pivoting/locking section (32), preferably at the pivot carrier element (18), and by a pivot recess (50, 52) open in a circumferential area being provided at the other assembly out of pivot carrier element (18) and pivoting/locking section (32), preferably at the pivoting/locking section (32) of the guiding path element (30) in association to each pivot projection (58, 60), for inserting a pivot projection (58, 60).

6. Device according to claim 5,
**characterized by** the at least one pivot projection (58, 60) for insertion into a pivot recess (50, 52) comprises in a relative insertion positioning between the pivoting/locking section (32) and the pivot carrier element (18) in at least one circumferential region a smaller cross section than in another circumferential section, wherein when the pivot projection (58, 60) is received in the pivot recess (50, 52) and when the pivoting/locking section (32) and the pivot carrier element (18) are pivoted in relation to each other, the at least one pivot projection (58, 60) cannot be moved out of the pivot recess (50, 52).

7. Device according to claim 5 or 6,
**characterized in that** two pivot projections (58, 60) extending substantially along a common pivot axis (S) are provided at the pivot carrier element (18) and **in that** the pivoting/locking section (32) of the guiding path element (30) is adapted fork-like with two fork end sections (46, 48) and comprises a pivot recess (50, 52) at each fork end section (46, 48).

8. Device according to claim 3 to 7,
**characterized by** at least one locking projection (68, 70) being provided at one assembly out of pivot carrier element (18) and pivoting/locking section (32), preferably at the pivot carrier element (18) and by a locking recess (76, 78) for inserting a locking projection (68, 70) open to the circumferential area being provided at the other assembly out of pivot carrier element (18) and pivoting/locking section (32), preferably at the pivoting/locking section (32) of the guiding path element (30), in association to each locking projection (68, 70).

9. Device according to claim 8,
**characterized in that** the at least one locking recess (68, 70) can be moved in the direction of a projection longitudinal axis (A) between a receiving position for insertion into a locking recess (76, 78) and a locking position, wherein in the locking position a locking portion (90, 92) of the locking projection (68, 70) engages a counter locking portion (94, 96) of the locking recess (76, 78) in such a way that the locking projection (68, 70) cannot be moved out of the locking recess (76, 78).

10. Device according to claim 9,
**characterized by** the at least one locking projection (68, 70) being biased in its locking position.

11. Device according to claim 9 or 10,
**characterized in that** two locking projections (68, 70) extending along a common projection longitudinal axis (A) are provided at the pivot carrier element (18) and **in that** two locking recess projections (72, 74) each comprising a locking recess (76, 78) are provided at the pivoting/locking section (32) of said guiding path element (30).

12. Device according to one of claims 1 to 11,
**characterized by** the optics carrier arrangement (28) comprising a guiding path section (34) which is or can be positioned at the proximal end (16) of said device body (12), with a first guiding structure (97), preferably a guiding groove (98) or a guiding surface (200), surrounding the longitudinal axis of the device body (L) in a ring-like manner, in one assembly out of guiding path section (34) and optics carrier (36), preferably the guiding path section (34), and with a second guiding structure (103), preferably a guiding projection (104) or a guiding ring (204), at the other assembly out of guiding path section (34) and optics carrier (36), preferably at the optics carrier (36), guided along the first guiding structure (97) for moving the optics carrier (36) on its movement path (B) about the longitudinal axis of the device body (L) along the first guiding structure (97).

13. Device according to one of claims 12,
**characterized by** the first guiding structure (97) comprising a guiding groove (98) and the second guiding structure (103) comprising a guiding projection (104), the guiding groove (98) being open from the proximal end section (16) of the device body (12) in the direction of the longitudinal axis of the device body (L).

14. Device according to claim 13,
**characterized in that** the guiding groove (98) comprises a groove undercut section (100) and **in that** an undercut engagement section (116) engaging the groove undercut section (100) is provided at the guiding projection (104).

15. Device according to claim 12,
**characterized by** the first guiding structure (97) comprising a guiding surface (200) and by the second guiding structure (103) comprising a guiding ring (204), the guiding ring (204) comprising a counter guiding surface (208) for abutment at the guiding surface (200).

16. Device according to claim 15,
**characterized by** one surface out of guiding surface (200) and counter guiding surface (208) being an inner circumferential surface and the other surface being an outer circumferential surface.

17. Device according to claim 15 or 16,
**characterized in that** a guiding depression (202) extending in the direction of the movement path (B) is provided in association to a surface out of guiding surface (200) and counter guiding surface (208) and **in that** a guiding projection (210) extending in the direction of the movement path (B) for engaging the guiding depression (206) is provided in association to the other surface.

18. Device according to one of claims 15 to 17,
**characterized by** the guiding ring (204) being radial-elastic.

19. Device according to one of claims 15 to 18,
**characterized in that** a further guiding surface (228) surrounding the longitudinal axis of the device body (L) in a ring-like manner is provided in the region of the proximal end (16) of the device body (12) and **in that** a further counter guiding surface (230) is provided at the guiding ring (204) for abutment (230) at the other guiding surface (228).

20. Device according to one of claims 12 to 19,
**characterized by** one assembly out of guiding path section (34) and optics carrier (36), preferably the optics carrier (36) comprising a fixing element (122) which can be pressed against the other assembly out of guiding path section (34) and optics carrier (36), preferably the guiding path section (34), for fixing the optics carrier (36) preferably in any position along the guiding groove (98) in relation to the guiding path section (34).

21. Device according to claim 20,
**characterized by** a fixing element (122) which can be pressed substantially from radially outside in relation to the longitudinal axis of the device body (L) against an abutment region at the guiding path section (34) being provided at the optics carrier (36).

22. Device according to claim 21,
**characterized in that** the fixing element (122) can be moved substantially radially in relation to the longitudinal axis of the device body (L) for realizing and releasing the fixation.

23. Device according to claim 20, 21 or 22,
**characterized by** a fixing element actuator (130, 138, 142) being associated to the fixing element (122) for adjusting the fixing element (122) between a fixing position and a release position.

24. Device according to claim 23,
**characterized in that** the fixing element actuator (130, 138, 142) comprises a first impact area (134) which can be moved with the first fixing element (122) and a second impact area (140) at an impact element (138) acting on the first impact area (134) and being moveable in relation to the first impact area (134) for movement with the fixing element (122), wherein at least one impact area (134, 140), preferably each impact area (134, 140), is formed wedge-like.

25. Device according to claim 24,
**characterized by** the first impact area (134) being displaceable with the fixing element (122) in a first displacement direction (R₁) and by the second impact area (140) being displaceable with the impact element (138) in a second displacement direction (R₂) which is angled in relation to the first displacement direction (R₁), preferably substantially orthogonal to the latter.

26. Device according to claim 24 or 25,
**characterized by** a thread drive (142) being associated to the impact element (138).

27. Device according to one of claims 24 to 26,
**characterized by** the first impact area (134) and the second impact area (140) being in form fit engagement.

28. Device according to claims 1 to 27,
**characterized by** an optics fixing section (148) for fixing an optical system (38) at the optics carrier (36) being provided at the optics carrier (36).

29. Device according to claim 28,
**characterized by** the optics fixing section (148) comprising a dovetail inner profile (150) or a dovetail outer profile (160), preferably a dovetail inner profile (150), as well as an optics fixing element (152) at the optics carrier (36) for establishing and releasing the fixation of the optical system (38) which can be moved in relation to the optics carrier (36).

## Revendications

1. Dispositif pour prévoir une ouverture d'accès, en particulier pour une opération de la colonne vertébrale, comprenant un corps de dispositif tubulaire (12) s'étendant le long de l'axe longitudinal du corps de dispositif (L) avec une extrémité distale (14) à positionner à l'intérieur d'un corps et une extrémité proximale (16) à positionner à l'extérieur d'un corps ainsi qu'un arrangement de support d'optique (28), où l'arrangement de support d'optique (28) est positionné ou peut être positionné dans la région de l'extrémité proximale (16) avec un support d'optique (36) mobile dans une voie de mouvement (B) autour de l'axe longitudinal du corps de dispositif (L),
**caractérisé par** l'arrangement de support d'optique (28) comprenant un membre de voie de guidage (30) supportant le support d'optique (36), avec une section de voie de guidage (34) qui, en pivotant autour d'un axe de pivotement (S), peut être positionnée dans une position opératoire dans la région de l'extrémité proximale (16) du corps de dispositif (12).

2. Dispositif selon la revendication 1,
**caractérisé par** la section de voie de guidage (34) étant adaptée de manière annulaire et comprenant une première structure de guidage (97) annulaire, de préférence une rainure de guidage (98) ou une surface de guidage (200), où une deuxième structure de guidage (103), de préférence une saillie de guidage (104) ou une bague de guidage (204), du support d'optique (36) est guidée le long de la première structure de guidage (98) annulaire pour déplacer le support d'optique (36) dans sa voie de mouvement (B) autour de l'axe longitudinal du corps de dispositif (L) et peut être fixée de préférence à chaque position le long de la première structure de guidage (98).

3. Dispositif selon la revendication 1 ou 2,
**caractérisé par** le membre de voie de guidage (30) comprenant une section de pivotement/d'arrêt (32) pour monter et arrêter le membre de voie de guidage (30) de manière pivotante dans la position opératoire à un membre de support pivotant (18) s'éloignant du corps de dispositif (12).

4. Dispositif selon la revendication 3,
**caractérisé en ce que** le membre de support pivotant (18) comprend une région de montage (20) entourant de préférence le corps de dispositif (12) d'une manière annulaire et terminant de manière solidaire avec l'extrémité proximale (16) du corps de dispositif (12) et une région de support (24) s'étendant de la région de montage (20).

5. Dispositif selon la revendication 3 ou 4,
**caractérisé par** au moins une saillie de pivotement (58, 60) étant prévue à un ensemble parmi le membre de support pivotant (18) et la section de pivotement/d'arrêt (32), de préférence au membre de support pivotant (18), et par une encoche de pivotement (50, 52) qui est ouverte dans une région circonférentielle étant prévue à l'autre ensemble parmi le support pivotant (18) et la section de pivotement/d'arrêt (32), de préférence à la section de pivotement/d'arrêt (32) du membre de voie de guidage (30), en association à chaque saillie de pivotement (58, 60) pour insérer une saillie de pivotement (58, 60).

6. Dispositif selon la revendication 5
**caractérisé en ce que** ladite au moins une saillie de pivotement (58, 60) pour l'insertion dans une encoche de pivotement (50, 52) comprend dans une position relative d'insertion entre la section de pivotement/d'arrêt (32) et le membre de support pivotant (18) dans au moins une région circonférentielle une section transversale qui est plus petite que dans une autre région circonférentielle, où lorsque la saillie de pivotement (58, 60) est reçue dans l'encoche de pivotement (50, 52) et lorsque la section de pivotement/d'arrêt (32) et le membre de support pivotant (18) sont pivotés l'un par rapport à l'autre, ladite au moins une saillie de pivotement (58, 60) ne peut pas être sortie de l'encoche de pivotement (50, 52).

7. Dispositif selon la revendication 5 ou 6,
**caractérisé par** deux saillies de pivotement (58, 60) qui s'étendent essentiellement le long d'un axe commun de pivotement (S) étant prévues au membre de support pivotant (18) et par la section de pivotement/d'arrêt (32) du membre de voie de guidage (30) étant adaptée en fourche avec deux sections d'extrémité de fourche (46, 48) et comprenant une encoche de pivotement (50, 52) à chaque section d'extrémité de fourche (46, 48).

8. Dispositif selon une des revendications 3 à 7,
**caractérisé par** au moins une saillie d'arrêt (68, 70) étant prévue à un ensemble parmi le membre de support pivotant (18) et la section de pivotement/d'arrêt (32), de préférence au membre de support pivotant (18), et par une encoche d'arrêt (76, 78) ouverte dans une région circonférentielle pour insérer une saillie d'arrêt (68, 70) étant prévue à l'autre ensemble parmi le membre de support pivotant (18) et la section de pivotement/d'arrêt (32), de préférence à la section de pivotement/d'arrêt (32), en association à chaque saillie d'arrêt (68, 70).

9. Dispositif selon la revendication 8,
**caractérisé en ce que** ladite au moins une saillie d'arrêt (68, 70) peut être déplacée dans le sens d'un axe longitudinal de saillie (A) entre une position de réception pour l'insertion dans une encoche d'arrêt (76, 78) et une position d'arrêt, où dans la position d'arrêt une section d'arrêt (90, 92) de la saillie d'arrêt (68, 70) vient en prise sur une contre-section d'arrêt (94, 96) de l'encoche d'arrêt (76, 78) de sorte que la saillie d'arrêt (68, 70) ne peut pas être sortie de l'encoche d'arrêt (76, 78).

10. Dispositif selon la revendication 9,
**caractérise par** ladite au moins une saillie d'arrêt (68, 70) étant précontrainte dans sa position d'arrêt.

11. Dispositif selon les revendications 9 ou 10,
**caractérisé par** deux saillies d'arrêt (68, 70) qui s'étendent le long d'un axe longitudinal de saillie (A) étant prévues au membre de support pivotant (18) et par deux saillies d'encoche d'arrêt (72, 74), chacune comprenant une encoche d'arrêt (76, 78), étant prévues à la section de pivotement/d'arrêt (32) du membre de voie de guidage (30).

12. Dispositif selon une des revendications 1 à 11,
**caractérisé par** l'arrangement de support d'optique (28) comprenant une section de voie de guidage (34) qui est ou peut être positionnée à l'extrémité proximal (16) du corps de dispositif (12), avec une première structure de guidage (97), de préférence une rainure de guidage (98) ou une surface de guidage (200), entourant l'axe longitudinal du corps de dispositif (L) de manière annulaire, dans un ensemble parmi la section de voie de guidage (34) et le support d'optique (36), de préférence la section de voie de guidage (34), et avec une deuxième structure de guidage (103), de préférence une saillie de guidage (104) ou une bague de guidage (204), à l'autre ensemble parmi la section de voie de guidage (34) et le support d'optique (36), de préférence au support d'optique (36), guidée le long de la première structure de guidage (97) pour déplacer le support d'optique (36) sur sa voie de mouvement (B) autour de l'axe longitudinal du corps de dispositif (L) le long de la première structure de guidage (97).

13. Dispositif selon la revendication 12,
**caractérisé par** la première structure de guidage (97) comprenant une rainure de guidage (98) et par la deuxième structure de guidage (103) comprenant une saillie de guidage (104), la rainure de guidage (98) étant ouverte de la section d'extrémité proximale (16) du corps de dispositif (12) dans le sens de l'axe longitudinal du corps de dispositif (L).

14. Dispositif selon la revendication 13,
**caractérisé par** la rainure de guidage (98) comprenant une section de contre-dépouille de rainure (100) et par une section d'engagement de contre-dépouille (116) venant en prise sur la section de contre-dépouille de rainure (100) étant prévue à la saillie de guidage (104).

15. Dispositif selon la revendication 12,
**caractérisé par** la première structure de guidage (97) comprenant une surface de guidage (200) et par la deuxième structure de guidage (103) comprenant une bague de guidage (204), la bague de guidage (204) comprenant une contre-surface de guidage (208) pour venir en butée contre la surface de guidage (200).

16. Dispositif selon la revendication 15,
**caractérisé par** une surface de surface de guidage (200) et de contre-surface de guidage (208) étant une surface intérieure circonférentielle et l'autre surface étant une surface extérieure circonférentielle.

17. Dispositif selon la revendication 15 ou 16,
**caractérisé par** une dépression de guidage (202) qui s'étend dans le sens de la voie de mouvement (B) étant prévue en association à une surface parmi la surface de guidage (200) et la contre-surface de guidage (208) et par une saillie de guidage (210) qui s'étend dans le sens de la voie de mouvement (B) pour venir en prise sur la dépression de guidage (206) étant prévue en association à l'autre surface.

18. Dispositif selon une des revendications 15 à 17,
**caractérisé par** la bague de guidage (204) étant radialement élastique.

19. Dispositif selon une des revendications 15 à 18,
**caractérisé par** une autre surface de guidage (228) qui entoure l'axe longitudinal du corps de dispositif (L) de manière annulaire étant prévue dans la région de l'extrémité proximale (16) du corps de dispositif (12) et par une autre contre-surface de guidage (230) étant prévue à la bague de guidage (204) pour reposer (230) contre l'autre surface de guidage (208).

20. Dispositif selon une des revendications 12 à 19,
**caractérisé par** un ensemble parmi la section de voie de guidage (34) et le support d'optique (36), de préférence le support d'optique (36) comprenant un membre de fixation (122) qui peut être pressé contre l'autre ensemble parmi la section de voie de guidage (34) et le support d'optique (36), de préférence contre la section de voie de guidage (34), pour fixer le support d'optique (36) de préférence dans toute position le long de la rainure de guidage (98) en relation à la section de voie de guidage (34).

21. Dispositif selon la revendication 20,
**caractérisé par** un membre de fixation (122) qui peut être pressé essentiellement radialement depuis l'extérieur relatif à l'axe longitudinal du corps de dispositif (L) contre une région de butée à la section de voie de guidage (34) étant prévu au support d'optique (36).

22. Dispositif selon la revendication 21,
**caractérisé en ce que** le membre de fixation (122) peut être déplacé essentiellement de manière radiale relatif à l'axe longitudinal du corps de dispositif (L) pour réaliser et résoudre la fixation.

23. Dispositif selon les revendications 20, 21 ou 22,
**caractérisé par** un actionneur de membre de fixation (130, 138, 142) étant associé à l'élément de fixation (122) pour ajuster le membre de fixation (122) entre une position de fixation et une position de libération.

24. Dispositif selon la revendication 23,
**caractérisé en ce que** l'actionneur de membre de fixation (130, 138, 142) comprend une première région d'application (134) qui peut être déplacée avec le premier membre de fixation (122) et une deuxième région d'application (140) attachée à un membre d'application (138) agissant sur la première région d'application (134) et pouvant être déplacée par rapport à la première région d'application (134) pour un déplacement avec le membre de fixation (122), où au moins une région d'application (134, 140), de préférence chaque région d'application (134, 140) est adaptée en forme de coin.

25. Dispositif selon la revendication 24
**caractérisé par** la première région d'application (134) pouvant être déplacée avec le membre de fixation (122) dans un premier sens de déplacement (R₁) et par la deuxième région d'application (140) pouvant être déplacée avec le membre d'application (138) dans un deuxième sens de déplacement (R₂) qui est en angle par rapport au premier sens de déplacement (R₁), de préférence essentiellement orthogonal au dernier.

26. Dispositif selon la revendication 24 ou 25,
**caractérisé par** un entraînement de filetage (142) étant associé au membre d'application (138).

27. Dispositif selon une des revendications 24 à 26,
**caractérisé par** la première région d'application (134) et la deuxième région d'application (140) étant en engagement solidaire.

28. Dispositif selon une des revendications 1 à 27,
**caractérisé par** une section de fixation d'optique (148) pour fixer un système optique (38) au support d'optique (36) étant prévue au support d'optique (36).

29. Dispositif selon la revendication 28,
**caractérisé par** la section de fixation d'optique (148) comprenant un profil intérieur en queue d'aronde (150) ou un profil extérieur en queue d'aronde (160), de préférence un profil intérieur en queue d'aronde (150) et un élément de fixation d'optique (152) au support d'optique (36) pour réaliser et résoudre la fixation du système optique (38) qui peut être déplacé par rapport au support d'optique (36).
